(19) Europäisches Patentamt
European Patent Office
Office européen des brevets

(11) Veröffentlichungsnummer: **0 115 472**
**A2**

# (12) EUROPÄISCHE PATENTANMELDUNG

(21) Anmeldenummer: **84810040.0**

(22) Anmeldetag: **23.01.84**

(51) Int. Cl.³: **C 07 C 103/52**
A 61 K 37/02

(30) Priorität: **27.01.83 CH 453/83**

(43) Veröffentlichungstag der Anmeldung:
**08.08.84 Patentblatt 84/32**

(84) Benannte Vertragsstaaten:
**AT BE CH DE FR GB IT LI LU NL SE**

(71) Anmelder: **CIBA-GEIGY AG**
**Postfach**
**CH-4002 Basel(CH)**

(72) Erfinder: **Bencze, William, Dr.**
**Scheltenstrasse 6**
**CH-4106 Therwil(CH)**

(72) Erfinder: **Kamber, Bruno, Dr.**
**Sonnenweg 9**
**CH-4144 Arlesheim(CH)**

(72) Erfinder: **Storni, Angelo, Dr.**
**Im Feuerbusch 3**
**CH-4310 Rheinfelden(CH)**

## (54) Pyrrolidinonderivate und Verfahren zu ihrer Herstellung.

(57) Die Erfindung betrifft Lactampeptide der Formel I,

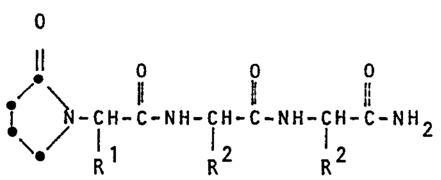

(I)

worin

$R^1$ Wasserstoff, $C_{1-4}$-Alkyl, Arylniederalkyl oder Aryl und

$R^2$ Wasserstoff, Niederalkyl oder Aryl bedeuten, wobei beide Reste $R^2$ die gleiche Bedeutung haben, und Salze von solchen Verbindungen mit mindestens einer salzbildenden Gruppe,

Verfahren und neue Zwischenprodukte zur Herstellung dieser Verbindungen, pharmazeutische Präparate, die diese Verbindungen enthalten, und ihre Verwendung zur Herstellung pharmazeutischer Präparate oder als pharmakologisch wirksame Verbindungen.

Diese Verbindungen können zur Behandlung zerebraler Leistungsinsuffizienz verwendet werden.

EP 0 115 472 A2

Croydon Printing Company Ltd.

## Pyrrolidinonderivate und Verfahren zu ihrer Herstellung

Die Erfindung betrifft Lactampeptide der Formel I,

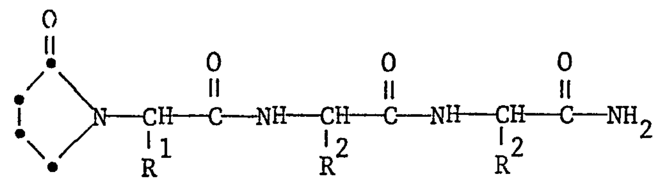

(I)

worin

$R^1$ Wasserstoff, $C_{1-4}$-Alkyl, Arylniederalkyl oder Aryl und
$R^2$ Wasserstoff, Niederalkyl oder Aryl bedeuten, wobei beide Reste $R^2$ die gleiche Bedeutung haben, und Salze von solchen Verbindungen mit mindestens einer salzbildenden Gruppe,

Verfahren und neue Zwischenprodukte zur Herstellung dieser Verbindungen, pharmazeutische Präparate, die diese Verbindungen enthalten, und ihre Verwendung zur Herstellung pharmazeutischer Präparate oder als pharmakologisch wirksame Verbindungen.

Das Präfix "Nieder" bezeichnet hier und im folgenden einen Rest mit 1-7, insbesondere 1-4 C-Atomen.

Aryl steht für einen unsubstituierten oder substituierten carbocyclischen aromatischen Rest. In erster Linie steht Aryl in dieser Anmeldung für unsubstituiertes oder substituiertes Phenyl, daneben auch für unsubstituiertes oder substituiertes Naphthyl.

Als Arylsubstituenten sind hauptsächlich zu nennen: Niederalkyl, Halogen und freies oder funktionell abgewandeltes Hydroxy, daneben auch freies oder verestertes Carboxy, Oxo oder unsubstituiertes oder substituiertes Phenyl.

Funktionell abgewandeltes Hydroxy ist verestertes oder verethertes Hydroxy.

Verestertes Hydroxy ist vorzugsweise durch eine organische, aber auch durch eine anorganische Säure verestertes Hydroxy, vor allem Acyloxy, daneben auch Sulfonyloxy oder Halogen. Acyloxy ist vorzugsweise gegebenenfalls substituiertes Hydrocarbylcarbonyloxy oder Hydrocarbyloxycarbonyloxy. Gegebenenfalls substituiertes Hydrocarbyl steht hier und im folgenden insbesondere für einen aliphatischen Rest mit 1-21, in erster Linie 1-11, vor allem 1-7 C-Atomen, einen carbocyclischen Rest, d.h. einen cycloaliphatischen Rest mit 3-8, insbesondere 5 oder 6, Ringgliedern und bis zu 21, vorzugsweise bis zu 11 C-Atomen, oder einen mono- oder bicyclischen aromatischen Rest mit 6 oder 10 Ringgliedern und bis zu 21, vorzugsweise bis zu 15 C-Atomen, oder einen entsprechenden carbocyclisch-aliphatischen Rest. Bei den obengenannten cycloaliphatischen beziehungsweise aromatischen Resten handelt es sich vorzugsweise um unsubstituierte oder substituierte Cycloalkylreste, insbesondere unsubstituierte oder durch Niederalkyl substituierte Cycloalkylreste, beziehungsweise um Phenylreste, z.B. Phenyl.

Sulfonyloxy ist insbesondere aromatisches, vornehmlich monocyclisches aromatisches, Sulfonyloxy mit höchstens 12 Kohlenstoffatomen, z.B. Toluolsulfonyloxy, oder niederaliphatisches, vorzugsweise Niederalkylsulfonyloxy.

Verethertes Hydroxy ist insbesondere gegebenenfalls substituiertes Hydrocarbyloxy.

Verestertes Carboxy ist insbesondere unsubstituiertes oder substituiertes Hydrocarbyloxycarbonyl, vor allem Niederalkoxycarbonyl.

Niederalkyl $R^2$ ist vorzugsweise Methyl, Isopropyl, 1-Methyl-propyl oder Isobutyl.

Die Konfiguration an gegebenenfalls vorhandenen Asymmetriezentren in einer Verbindung der Formel I kann unabhängig voneinander (D), (L) oder (D,L) sein, ist jedoch an den <u>C</u>-$R^2$ -Atomen vorzugsweise (L).

Die vor- und nachstehend verwendeten Allgemeinbegriffe haben im Rahmen der vorliegenden Beschreibung vorzugsweise die folgenden Bedeutungen:

Niederalkyl ist vor allem Methyl, Ethyl, n-Propyl, Isopropyl, n-Butyl, und Isobutyl, daneben z.B. auch sek. Butyl oder tert. Butyl, ferner n-Pentyl, Neopentyl, n-Hexyl oder n-Heptyl.
Halogen ist insbesondere Fluor oder Chlor, kann jedoch auch für Brom oder Jod stehen.
Cycloalkyl ist z.B. Cyclohexyl oder Cyclopentyl.

Die Erfindung betrifft auch die insbesondere als Zwischenprodukte verwendbaren Verbindungen der Formel I mit geschützten funktionellen Gruppen, insbesondere geschütztem Hydroxy oder Carboxy.

Schutzgruppen, ihre Einführung und Abspaltung sind beispielsweise beschrieben in "Protective Groups in Organic Chemistry", Plenum Press, London, New York 1973, und in "Methoden der organischen Chemie", Houben-Weyl, 4. Auflage, Bd. 15/1, Georg-Thieme-Verlag, Stuttgart 1974. Charakteristisch für Schutzgruppen ist, dass sie leicht, d.h. ohne dass unerwünschte Nebenreaktionen stattfinden, z.B. solvolytisch, reduktiv, photolytisch oder auch unter physiologischen Bedingungen abspaltbar sind.

Hydroxyschutzgruppen sind z.B. Acylreste, wie gegebenenfalls, z.B. durch Halogen, substituiertes Niederalkanoyl, wie 2,2-Dichloracetyl,

oder Acylreste von Kohlensäurehalbestern, insbesondere tert.-Butyloxycarbonyl, gegebenenfalls substituiertes Benzyloxycarbonyl, z.B. 4-Nitro-benzyloxycarbonyl, oder Diphenylmethoxycarbonyl, oder 2-Halogen-niederalkoxycarbonyl, wie 2,2,2-Trichlorethoxycarbonyl, ferner Trityl oder Formyl, oder organische Silyl- oder Stannylreste, ferner leicht abspaltbare verethernde Gruppen, wie tert.-Niederalkyl, z.B. tert.-Butyl, 2-oxa- oder 2-thia-aliphatische oder -cycloaliphatische Kohlenwasserstoffreste, in erster Linie 1-Niederalkoxyniederalkyl oder 1-Niederalkylthio-niederalkyl, z.B. Methoxymethyl, 1-Methoxyethyl, 1-Ethoxy-ethyl, 1-Methylthiomethyl, 1-Methylthioethyl oder 1-Ethylthioethyl, oder 2-Oxa- oder 2-Thiacycloalkyl mit 5-6 Ringatomen, z.B. Tetrahydrofuryl oder 2-Tetrahydropyranyl oder entsprechende Thiaanaloge, sowie gegebenenfalls substituiertes 1-Phenylniederalkyl, wie gegebenenfalls substituiertes Benzyl oder Diphenylmethyl, wobei als Substituenten der Phenylreste z.B. Halogen, wie Chlor, Niederalkoxy, wie Methoxy und/oder Nitro in Frage kommen.

Carboxylgruppen sind üblicherweise in veresterter Form geschützt, wobei solche Estergruppierungen unter schonenden Bedingungen leicht spaltbar sind. In dieser Art geschützte Carboxylgruppen enthalten als veresternde Gruppen in erster Linie in 1-Stellung verzweigte oder in 1- oder 2-Stellung geeignet substituierte Niederalkylgruppen. Bevorzugte, in veresterter Form vorliegende Carboxylgruppen sind u.a. tert.-Niederalkoxycarbonyl, z.B. tert.-Butyloxycarbonyl, Arylmethoxycarbonyl mit einem oder zwei Arylresten, wobei diese gegebenenfalls z.B. durch Niederalkyl, wie tert.-Niederalkyl, z.B. tert.-Butyl, Niederalkoxy, wie Methoxy, Hydroxy, Halogen, z.B. Chlor, und/oder Nitro, mono- oder polysubstituierte Phenylreste darstellen, wie gegebenenfalls, z.B. wie oben erwähnt, substituiertes Benzyloxycarbonyl, z.B. 4-Methoxybenzyloxycarbonyl, oder 4-Nitrobenzyloxycarbonyl, oder gegebenenfalls, z.B. wie oben erwähnt, substituiertes Diphenylmethoxycarbonyl, z.B. Diphenylmethoxycarbonyl oder Di-(4-methoxyphenyl)-methoxycarbonyl, 1-Niederalkoxyniederalkoxycarbonyl, wie Methoxymethoxycarbonyl, 1-Methoxyethoxycarbonyl oder 1-Ethoxymethoxycarbonyl, 1-Niederalkylthioniederalkoxycarbonyl, wie 1-Methylthiomethoxycarbonyl oder 1-Ethylthioethoxycarbonyl, Aroylmethoxycarbonyl, worin die Aroylgruppe gegebenenfalls, z.B. durch Halogen, wie Brom, substituiertes Benzoyl darstellt, z.B. Phenacyloxycarbonyl, 2-Halogenniederalkoxycarbonyl, z.B. 2,2,2-Trichlorethoxycarbonyl, 2-Bromethoxycarbonyl oder 2-Jodethoxycarbonyl, oder 2-(trisubstituiertes Silyl)-ethoxycarbonyl, worin die Substituenten unabhängig voneinander je einen gegebenenfalls substituierten, z.B. durch Niederalkyl, Niederalkoxy, Aryl, Halogen, und/oder Nitro substituierten, aliphatischen, araliphatischen, cycloaliphatischen oder aromatischen Kohlenwasserstoffrest, wie entsprechendes, gegebenenfalls substituiertes Niederalkyl, Phenylniederalkyl, Cycloalkyl oder Phenyl, bedeuten, z.B. 2-Triniederalkylsilylethoxycarbonyl, wie 2-Trimethylsilylethoxycarbonyl oder 2-(Di-n-butyl-methyl-silyl)-ethoxycarbonyl, oder 2-Triarylsilylethoxycarbonyl, wie 2-Triphenylsilylethoxycarbonyl. Die oben und im folgenden erwähnten organischen Silyl- oder Stannylreste enthalten vorzugsweise Niederalkyl, insbesondere Methyl, als Substituenten der Silizium- oder Zinn-Atome. Entsprechende Silyl- oder Stannylgruppen sind in erster Linie Triniederalkylsilyl, insbesondere Trimethylsilyl, ferner Dimethyl-tert.-butyl-silyl, oder entsprechend substituiertes Stannyl, z.B. Tri-n-butylstannyl.

Bevorzugte geschützte Carboxylgruppen sind tert.-Niederalkoxycarbonyl, wie tert.-Butoxycarbonyl, und in erster Linie gegebenenfalls, z.B. wie oben erwähnt, substituiertes Benzyloxycarbonyl, wie 4-Nitrobenzyloxycarbonyl, oder Diphenylmethoxycarbonyl, vor allem 2-(Trimethylsilyl)-ethoxycarbonyl.

Eine geschützte Aminogruppe, z.B. in einem Ausgangsmaterial zur Herstellung der Verbindungen der Formel I, kann z.B. in Form einer leicht spaltbaren Acylamino-, Arylmethylamino-, verätherten Mercaptoamino-, 2-Acylniederalk-1-en-1-yl-amino-, Silyl- oder Stannylaminogruppe oder als Azidogruppe vorliegen.

In einer entsprechenden Acylaminogruppe ist Acyl beispielsweise der Acylrest einer organischen Carbonsäure mit z.B. bis zu 18 Kohlenstoffatomen, insbesondere einer gegebenenfalls, z.B. durch Halogen oder Aryl, substituierten Alkancarbonsäure oder gegebenenfalls, z.B. durch Halogen, Niederalkoxy oder Nitro, substituierten Benzoesäure, oder eines Kohlensäurehalbesters. Solche Acylgruppen sind beispielsweise Niederalkanoyl, wie Formyl, Acetyl, oder Propionyl, Halogenniederalkanoyl, wie 2-Halogenacetyl, insbesondere 2-Chlor-, 2-Brom-, 2-Jod-, 2,2,2-Trifluor- oder 2,2,2-Trichloracetyl, gegebenenfalls, z.B. durch Halogen, Niederalkoxy oder Nitro substituiertes Benzoyl, z.B. Benzoyl, 4-Chlorbenzoyl, 4-Methoxybenzoyl oder 4-Nitrobenzoyl, oder in 1-Stellung der Niederalkylrestes verzweigtes oder in 1- oder 2-Stellung geeignet substituiertes Niederalkoxycarbonyl, insbesondere tert.-Niederalkoxycarbonyl, z.B. tert.-Butyloxycarbonyl, Arylmethoxycarbonyl mit einem oder zwei Arylresten, die vorzugsweise gegebenenfalls, z.B. durch Niederalkyl, insbesondere tert.Niederalkyl, wie tert.Butyl, Niederalkoxy, wie Methoxy, Hydroxy, Halogen, z.B. Chlor, und/oder Nitro, mono- oder polysubstituiertes Phenyl darstellen, wie gegebenenfalls substituiertes Benzyloxycarbonyl, z.B. 4-Nitro-benzyloxycarbonyl, oder substituiertes Diphenylmethoxycarbonyl, z.B. Benzhydryloxycarbonyl oder Di-(4-methoxyphenyl)-methoxycarbonyl, Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl, 2-Halogen-niederalkoxycarbonyl, z.B. 2,2,2-Trichlorethoxycarbonyl, 2-Bromethoxycarbonyl oder 2-Jodethoxycarbonyl, oder 2-(trisubstituiertes Silyl)-ethoxycarbonyl, worin die Substituenten unabhängig voneinander je einen gegebenenfalls substituierten, z.B. durch Niederalkyl, Niederalkoxy, Aryl, Halogen oder Nitro substituierten, aliphatischen, araliphatischen, cycloaliphatischen oder aromatischen Kohlenwasserstoffrest mit z.B. bis zu 15 C-Atomen, wie entsprechendes, gegebenenfalls substituiertes Niederalkyl, Phenylniederalkyl, Cycloalkyl oder Phenyl, bedeuten, z.B. 2-Triniederalkylsilylethoxycarbonyl, wie 2-Trimethylsilylethoxycarbonyl oder 2-(Di-

n-butyl-methyl-silyl)-ethoxycarbonyl, oder 2-Triarylsilylethoxycarbonyl, wie 2-Triphenylsilylethoxycarbonyl.

Weitere, als Aminoschutzgruppen in Frage kommende Acylreste sind auch entsprechende Reste organischer Phosphor-, Phosphon- oder Phosphinsäuren, wie Diniederalkylphosphoryl, z.B. Dimethylphosphoryl, Diethylphosphoryl, Di-n-propylphosphoryl oder Diisopropylphosphoryl, Dicycloalkylphosphoryl, z.B. Dicyclohexylphosphoryl, gegebenenfalls substituiertes Diphenylphosphoryl, z.B. Diphenylphosphoryl, gegebenenfalls, z.B. durch Nitro substituiertes Di-(phenylniederalkyl)-phosphoryl, z.B. Dibenzylphosphoryl oder Di-(4-nitrobenzyl)-phosphoryl, gegebenenfalls substituiertes Phenyloxy-phenyl-phosphonyl, z.B. Phenyloxyphenyl-phosphonyl, Diniederalkylphosphinyl, z.B. Diäthylphosphinyl, oder gegebenenfalls substituiertes Diphenylphosphinyl, z.B. Diphenylphosphinyl.

In einer Arylmethylaminogruppe, die eine Mono-, Di- oder insbesondere Triarylmethylamino darstellt, sind die Arylreste insbesondere gegebenenfalls substituierte Phenylreste. Solche Gruppen sind beispielsweise Benzyl-, Diphenylmethyl- und insbesondere Tritylamino.

Eine verätherte Mercaptogruppe in einer mit einem solchen Rest geschützten Aminogruppe ist in erster Linie Arylthio oder Arylniederalkylthio, worin Aryl insbesondere gegebenenfalls, z.B. durch Niederalkyl, wie Methyl oder tert.-Butyl, Niederalkoxy, wie Methoxy, Halogen, wie Chlor, und/oder Nitro substituiertes Phenyl ist. Eine entsprechende Aminoschutzgruppe ist z.B. 4-Nitrophenylthio.

In einem als Aminoschutzgruppe verwendbaren 2-Acyl-niederalk-1-en-1-yl-rest ist Acyl z.B. der entsprechende Rest einer Niederalkancarbonsäure, einer gegebenenfalls, z.B. durch Niederalkyl, wie Methyl oder tert.-Butyl, Niederalkoxy, wie Methoxy, Halogen, wie Chlor, und/oder Nitro substituierten Benzoesäure, oder insbesondere eines Kohlensäurehalbesters, wie eines Kohlensäure-niederalkylhalbesters.

Entsprechende Schutzgruppen sind in erster Linie 1-Niederalkanoyl-prop-1-en-2-yl, z.B. 1-Acetyl-prop-1-en-2-yl, oder 1-Niederalkoxy-carbonyl-prop-1-en-2-yl, z.B. 1-Aethoxycarbonyl-prop-1-en-2-yl.

Eine Aminogruppe kann auch in protonierter Form geschützt werden; als entsprechende Anionen kommen in erster Linie diejenigen von starken anorganischen Säuren, wie von Halogenwasserstoffsäuren, z.B. das Chlor- oder Bromanion, oder von organischen Sulfonsäuren, wie p-Toluolsulfonsäure, in Frage.

Bevorzugte Aminoschutzgruppen sind Acylreste von Kohlensäurehalbestern, insbesondere tert.-Butyloxycarbonyl, gegebenenfalls, z.B. wie angegeben, substituiertes Benzyloxycarbonyl, z.B. 4-Nitro-benzyloxycarbonyl, oder Diphenylmethoxycarbonyl, oder 2-Halogen-niederalkoxycarbonyl, wie 2,2,2-Trichloräthoxycarbonyl, ferner Trityl oder Formyl.

Salze der erfindungsgemässen Verbindungen mit salzbildenden Gruppen sind in erster Linie pharmazeutisch verwendbare, nicht-toxische Salze, wie diejenigen von Verbindungen der Formel I mit sauren Gruppen, z.B. mit einer freien Carboxylgruppe. Solche Salze sind in erster Linie Metall- oder Ammoniumsalze, wie Alkalimetall- und Erdalkalimetall-, z.B. Natrium-, Kalium-, Magnesium- oder Calciumsalze, sowie Ammoniumsalze mit Ammoniak oder geeigneten organischen Aminen, wobei in erster Linie aliphatische, cycloaliphatische, cycloaliphatisch-aliphatische oder araliphatische primäre, sekundäre oder tertiäre Mono-, Di- oder Polyamine, sowie heterocyclische Basen für die Salzbildung in Frage kommen, wie Niederalkylamine, z.B. Triethylamin, Hydroxyniederalkylamine, z.B. 2-Hydroxyethylamin, Bis-(2-hydroxyethyl)-amin, 2-Hydroxyethyldiethyl-amin oder Tri-(2-hydroxyethyl)-amin, basische aliphatische Ester von Carbonsäuren, z.B. 4-Aminobenzoesäure-2-diethylaminoethylester, Niederalkylenamine, z.B. 1-Ethyl-piperidin, Cycloalkylamine, z.B. Dicyclohexylamin, oder Benzylamine, z.B. N,N'-Dibenzylethylendiamin, ferner Basen vom Pyridintyp, z.B. Pyridin, Collidin oder Chinolin.

Zur Isolierung oder Reinigung können auch pharmazeutisch ungeeignete Salze Verwendung finden. Zur therapeutischen Anwendung gelangen nur die pharmazeutisch verwendbaren, nicht-toxischen Salze, die deshalb bevorzugt werden.

Die Verbindungen der Formel I, worin die funktionellen Gruppen in freier, d.h. nicht in geschützter Form vorliegen, wobei jedoch Carboxyl- und Hydroxylgruppen gegebenenfalls in physiologisch spaltbarer, veresterter Form vorliegen, und ihre pharmazeutisch verwendbaren, nicht-toxischen Salze sind wertvolle, pharmakologisch wirksame Substanzen. Insbesondere bewirken sie einen Schutz vor der amnesiogenen Wirkung von cerebralem Elektroschock und eine Verbesserung der Gedächtnisleistung. Befunde der genannten Art können in folgenden Lerntests ermittelt werden:
Einer dieser Lerntests ist ein sogenannter two-compartment passive avoidance test, modifiziert von Jarvik und Koop, Psychol. Rep. 21, 221-224 (1967).

Die Testvorrichtung besteht aus einer grossen Box (35 x 20 x 10 cm), die durch eine Schiebetür mit einer kleinen Box (10 x 10 x 18 cm) verbunden ist. Während die kleine Box durch eine 100 Watt Lampe von oben hell erleuchtet wird, ist die grosse Box dunkel. Der Boden beider Abteile besteht aus einem elektrisch leitenden Gitterrost dessen Gitterstäbe (6 mm Durchmesser) in einem Abstand von jeweils 13 mm angeordnet sind. Zum Training werden Gruppen von jeweils 10 männlichen Mäusen mit einem Körpergewicht von 20-22 g einzeln in die hell erleuchtete kleine Box gesetzt. Da Mäuse eine Spontanpräferenz für Dunkel haben, gehen die meisten innerhalb von 30 Sekunden ins dunkle Abteil. Sobald sie dieses vollständig betreten haben, wird die Schiebetür geschlossen und ein Fussschock (1 mA, 50 Hz, 5 Sekunden) verabreicht. Die Tiere werden darauf sofort aus dem Versuchsapparat entfernt.

Zur Prüfung des Lerneffektes (Retest) werden die Mäuse nach 24 Stunden wiederum einzeln ins helle Abteil gesetzt und die Zeitspanne bis sie

sich ganz im dunklen Abteil befinden, gemessen. Ueblicherweise bleiben nun die meisten Tiere über die ganze Beobachtungszeit von 150 Sekunden im hellen Abteil. Der Lerneffekt wird weitgehend aufgehoben bzw. die Erinnerung an den Fussschock mindestens partiell ausgelöscht, wenn als amnesiogene Behandlung unmittelbar anschliessend an den Fussschock des Lerndurchgangs eine temporale Elektroschockbehandlung angeschlossen wird. Parameter des Elektroschocks: 50 mA, 0,4 Sekunden, 50 Hz , 0,6 Millisekunden (Pulsdauer).

Zur Prüfung und zum Vergleich ihrer Schutzwirkung gegenüber der amnesiogenen Wirkung des Elektroschocks werden die Testsubstanzen 30 Minuten vor dem Lerndurchgang in Dosen von 0,1 mg/kg, 1 mg/kg, 10 mg/kg und 100 mg/kg (für jede Dosis werden 10 Mäuse verwendet) als Lösung in physiologischer NaCl-Lösung oder physiologische Kochsalzlösung alleine (= Placebo) intraperitoneal verabreicht und die Tiere unmittelbar nach dem Lerndurchgang der Elektroschockbehandlung unterzogen. 24 Stunden später wird anhand der Verweildauer in der beleuchteten Box das Ausmass des noch erhalten gebliebenen Lerneffektes gemessen und mit demjenigen der anderen Testsubstanzen wie auch von Kontrolltieren mit alleiniger Verabreichung des jeweils verwendeten Lösungsmittels und Training ohne, wie auch solchen mit anschliessender Elektroschockbehandlung, verglichen. Eine mit dem Placebo behandelte Gruppe ohne Elektroschockbehandlung dient zur Kontrolle des Lernens im Vermeidungstest, eine zweite mit Placebo behandelte Kontrollgruppe mit anschliessender Elektroschockbehandlung dient zur Ueberwachung des Ausmasses der amnesiogenen Wirkung des Elektroschocks.

Die oben erwähnten Wirkungen lassen sich beispielsweise nach intraperitonealer Verabreichung von N-[(2-Oxo-1-pyrrolidinyl)-acetyl]-glycyl-glycinamid in einem Dosisbereich von 0.1 bis 100 mg/kg nachweiweisen.

Zur unmittelbaren Messung der Verbesserung der Gedächtnisleistung wird ein anderer passiver Vermeidungstest (step down passive avoidance test, Psychopharmacol. 63, 297-300 [1979]) verwendet:

Die Testvorrichtung besteht aus einer mit Tageslicht normal beleuchteten Box (50 x 50 x 50 cm) mit einem elektrisch leitenden Gitterrost, dessen Gitterstäbe (6 mm Durchmesser) in einem Abstand von jeweils 13 mm angeordnet sind. In der Mitte des Gitterrostes ist eine hölzerne Plattform (10 mm Höhe, 67 mm Durchmesser) angebracht, welche von einer Plastikröhre (20 cm hoch, 68 mm innerer Durchmesser) umgeben ist. Zur Durchführung des Trainings werden Gruppen von jeweils 30 männlichen Mäusen mit einem Körpergewicht von 20-22g verwendet, indem jeweils ein Tier auf die von der Plastikröhre umgebene Plattform gesetzt, nach 10 Sekunden die Plastikröhre entfernt und die Zeitdauer gemessen wird, welche das Tier zum Hinabsteigen und Berühren des Gitterrostes mit allen 4 Pfoten benötigt. Wenn alle 4 Pfoten den Gitterrost berühren, wird ein Fussschock (1 mA, 50 Hz, 1 Sekunde) verabreicht. Innerhalb von 10 Sekunden nach Verabreichung des Fussschocks werden die Testsubstanzen in Dosen von 3 mg/kg, 10 mg/kg und 30 mg/kg (für jede Dosis werden 30 Mäuse verwendet) als Lösung in physiologischer NaCl-Lösung oder deren Lösungsmittel (physiologische Natriumchloridlösung = Placebo) intraperitoneal verabreicht. 24 Stunden später wird anhand der Verweildauer jedes einzelnen Tieres auf der Plattform das Ausmass der Verbesserung oder Verschlechterung des Lerneffekts im Vergleich zu den Tieren, denen zur Kontrolle lediglich das Lösungsmittel appliziert worden war, ermittelt. So lässt sich eine Verbesserung der Gedächtnisleistung beispielsweise schon nach i.p. Verabreichung von 10 mg/kg N-[(2-Oxo-1-pyrrolidinyl)-acetyl]-glycyl-glycinamid zeigen.

Aufgrund dieser Eigenschaften erscheinen die neuen Verbindungen zur Behandlung von zerebraler Leistungsinsuffizienz, insbesondere von Gedächtnisstörungen verschiedener Genese, wie senile Demenz, Multiinfarkt-Demenz oder Demenz vom Alzheimer-Typ, ferner von Folgeerscheinungen von Hirntraumen oder Apoplexie geeignet.

Die neuen Verbindungen besitzen ausserdem eine ausgeprägte tumorhemmende Wirkung. Sie hemmen nicht nur das Wachstum der Tumoren und verringern die Tumoranzahl, sondern sie erhöhen auch die Ueberlebenszeit. Dabei liegen zwei Vorteile der erfindungsgemässen Verbindungen gegenüber herkömmlichen cytostatischen oder cytotoxischen Drogen in der guten Verträglichkeit und darin begründet, dass sie als Nootropika die Lebensqualität der Tumorpatienten nicht mindern, sondern, im Gegenteil, diesen Patienten bei der Ueberwindung ihrer psychischen Probleme helfen. Die erfindungsgemässen Verbindungen können somit auch zur Therapie von Tumorerkrankungen, z.B. des Respirationstraktes, bei Warmblütern einschliesslich des Menschen verwendet werden.

Die tumorhemmende Wirkung der neuen Verbindungen kann z.B. durch folgenden Versuch gezeigt werden:

Bei syrischen Hamstern erzeugt man durch Applikation von Diethylnitrosamin in den Magen papilläre, epidermoide und adenocarcinomatöse Tumoren des Kehlkopfes, der Luftröhre und der Lungen [G. Papadopoulo und K.H. Schmidt-Ruppin, Oncology 33, 40-43 (1976)]. Ein Teil dieser an Tumoren erkrankten Hamster wird mit einer Verbindung der Formel I (z.B. 4 Wochen lang jeweils zweimal täglich an 5 Tagen pro Woche in einer Einzeldosis von je 20 mg/kg), der restliche Teil der Hamster mit einem Placebo behandelt. Drei oder 4 Tage nach dem Ende der Behandlung werden sämtliche Hamster getötet. Der Respirationstrakt wird herausgeschnitten und nach geeigneter Präparation auf die Grösse, Anzahl und Art der darin enthaltenen Tumoren mikroskopisch untersucht. Dabei findet man, dass die Grösse und Anzahl der Tumoren bei den mit einer Verbindung der Formel I behandelten Hamstern im Vergleich zu den unbehandelten Hamstern signifikant geringer ist.

Insbesondere betrifft die Erfindung Verbindungen der Formel I, worin $R^1$ Wasserstoff, $C_{1-4}$-Alkyl, durch einen unsubstituierten oder substituierten Phenyl- oder Naphthylrest substituiertes Niederalkyl oder einen unsubstituierten oder substituierten Phenylrest und $R^2$ Wasserstoff, Niederalkyl oder einen Phenylrest bedeuten, und Salze von solchen Verbindungen mit mindestens einer salzbildenden Gruppe.

In erster Linie betrifft die Erfindung Verbindungen der Formel I, worin $R^1$ Wasserstoff, unsubstituiertes oder durch Phenyl substituiertes $C_{1-4}$-Alkyl oder Phenyl und $R^2$ Wasserstoff, $C_{1-4}$-Alkyl, Phenyl oder 4-Hydroxy-phenyl bedeuten.

Hauptsächlich betrifft die Erfindung Verbindungen der Formel I, worin $R^1$ und $R^2$ unabhängig voneinander Wasserstoff oder $C_{1-4}$-Alkyl bedeuten.

Vor allem betrifft die Erfindung Verbindungen der Formel I, worin $R^1$ Wasserstoff oder $C_{1-4}$-Alkyl und $R^2$ Wasserstoff, Methyl, Ethyl, 2-Propyl, 2-Butyl oder 2-Methyl-propyl bedeuten.

Besonders betrifft die Erfindung die obengenannten Verbindungen der Formel I, worin $R^1$ Wasserstoff bedeutet und die an asymmetrisch substituierten $\underline{C}$-$R^2$-Atomen die (L)-Konfiguration aufweisen.

In allererster Linie betrifft die Erfindung N-[(2-Oxo-1-pyrrolidinyl)-acetyl]-(L)-alanyl-(L)-alaninamid und besonders N-[(2-Oxo-1-pyrrolidinyl)-acetyl]-glycyl-glycinamid.

Die erfindungsgemässen Verbindungen der Formel I und Salze von solchen Verbindungen mit mindestens einer salzbildenden Gruppe werden nach an sich bekannten Verfahren erhalten.

Verbindungen der Formel I und Salze von solchen Verbindungen, die mindestens eine salzbildende Gruppe enthalten, werden beispielsweise hergestellt, indem man

a) eine Verbindung der Formel II,

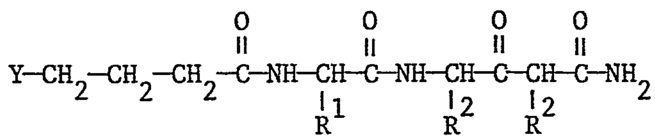 (II)

worin Y eine nucleophile Abgangsgruppe bedeutet und die übrigen Substituenten die obengenannten Bedeutungen haben, mit der Massgabe, dass in den Resten $R^1$ und $R^2$ gegebenenfalls vorhandene funktionelle Gruppen gegebenenfalls durch leicht abspaltbare Schutzgruppen geschützt sind, intramolekular unter Ausbildung des Pyrrolidonringes zyklisiert, oder

b) eine Verbindung der Formel III,

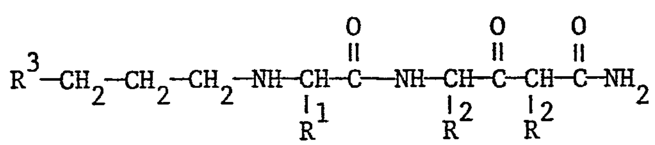 (III)

worin $R^3$ eine Carboxylgruppe oder ein aktiviertes Derivat derselben bedeutet und auch die an der Reaktion teilnehmende Aminogruppe in aktivierter Form vorliegen kann und die übrigen Substituenten die obengenannten Bedeutungen haben, mit der Massgabe, dass in den Resten $R^1$ und $R^2$ gegebenenfalls vorhandene funktionelle Gruppen gegebenenfalls durch leicht abspaltbare Schutzgruppen geschützt sind, intramolekular unter Ausbildung des Pyrrolidonringes zyklisiert oder

c) 2-Oxo-pyrrolidin der Formel IV

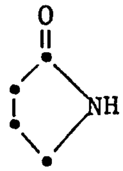

(IV)

oder eine reaktionsfähige Form dieser Verbindung mit einer Verbindung der Formel V,

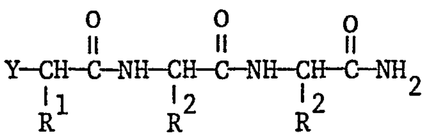

(V)

worin Y eine nucleophile Abgangsgruppe bedeutet und die übrigen Substituenten die obengenannten Bedeutungen haben, mit der Massgabe, dass in den Resten $R^1$ und $R^2$ vorhandene funktionelle Gruppen gegebenenfalls durch leicht abspaltbare Schutzgruppen geschützt sind, umsetzt, oder

d) eine Verbindung der Formel VI,

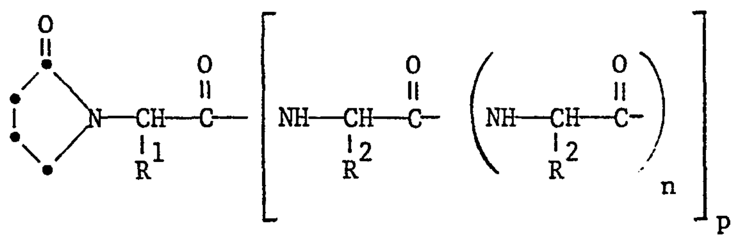

(VI)

worin n und p unabhängig voneinander die Zahl 0 oder 1 bedeuten, und die übrigen Substituenten die obengenannten Bedeutungen haben, mit der Massgabe, dass in den Resten $R^1$ und $R^2$ gegebenenfalls vorhandene funktionelle Gruppen gegebenenfalls durch leicht abspaltbare Schutzgruppen geschützt sind, oder ein reaktionsfähiges Carbonsäurederivat der Verbindung der Formel VI mit einer Verbindung der Formel VII,

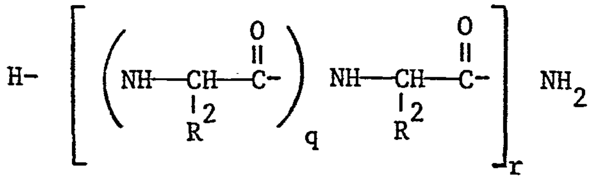

(VII)

worin q und r unabhängig voneinander für die Zahl 0 oder 1 stehen, mit der Massgabe, dass r und q jeweils für 1 stehen, wenn im Reaktionspartner der Formel VI p für 0 steht, oder dass r für 1 und q für 0 stehen, wenn p für 1 und n für 0 stehen, oder dass r für 0 steht, wenn p und n jeweils für 1 stehen, und worin die übrigen Substituenten die obengenannten Bedeutungen haben, mit der Massgabe, dass im Rest $R^2$ gegebenenfalls vorhandene funktionelle Gruppen gegebenenfalls durch leicht abspaltbare Schutzgruppen geschützt sind, oder ein Derivat der Verbindung der Formel VII, in dem eine der NH-Gruppen in reaktionsfähiger Form vorliegt, umsetzt, oder

e) die Cyanogruppe in einer Verbindung der Formel VIII ,

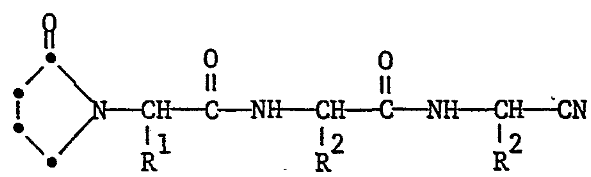

(VIII)

worin alle Substituenten die obengenannten Bedeutungen haben, in eine Amidgruppe überführt, oder

f) die Oximgruppe in einer Verbindung der Formel IX,

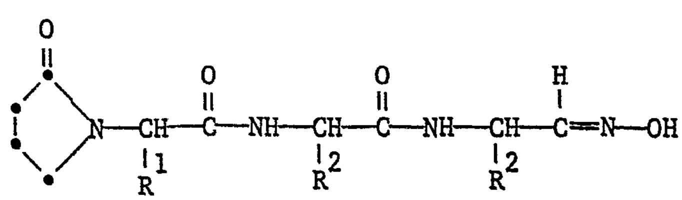

(IX)

worin die Substituenten die obengenannten Bedeutungen haben, durch eine Beckmann-Umlagerung in eine Amidgruppe überführt, oder

g) in einer Verbindung der Formel I, die mindestens eine freie Hydroxy- oder Carboxygruppe aufweist, freies Hydroxy verestert oder verethert oder freies Carboxy verestert und nach Ausführung der unter a) bis f) beschriebenen Verfahren gegebenenfalls vorhandene Schutzgruppen abspaltet und, wenn erwünscht, erhaltene Stereoisomerengemische auftrennt, und, wenn erwünscht, eine erhaltene Verbindung mit mindestens einer salzbildenden Gruppe in ein Salz oder ein erhaltenes Salz in die freie Verbindung überführt.

Verfahren a) (intramolekulare Zyklisierung einer Verbindung der Formel II): Eine nucleophile Abgangsgruppe Y ist insbesondere eine mit geeigneten Säuren veresterte Hydroxygruppe, z.B. ein Sulfonat, wie Mesylat oder Tosylat, vor allem Halogen, wie Brom oder Jod, besonders aber Chlor.

Die Reaktion wird mit Hilfe einer Base durchgeführt, wobei man vorzugsweise äquimolare Mengen dieser Base verwendet. Als Basen verwendet man insbesondere z.B. Metallhydroxide, -carbonate oder -alkoholate, wie Alkalimetall- oder Erdalkalimetallhydroxide oder -alkoholate, z.B. Natrium- oder Kaliumhydroxid oder Natrium-tert.-butylat, oder Alkalimetallcarbonate oder Salze, insbesondere Alkalimetallsalze, sekundärer Amide, z.B. 2-Oxo-pyrrolidin-natrium, oder starkbasische Ionenaustauscher, z.B. Dowex 1 (eingetragenes Warenzeichen), daneben auch Hydride oder Amide, z.B. Alkalimetallhydride oder -amide, wie Natriumhydrid oder -amid oder Lithiumdiisopropylamid.

Die Ringschlussreaktion wird vorzugsweise in einem inerten organischen Lösungsmittel, wenn erwünscht oder notwendig, unter Kühlen oder Erwärmen durchgeführt, z.B. in einem Temperaturbereich von etwa -80°C bis etwa +150°C, hauptsächlich zwischen 0°C und 100°C.

Generell sind die Reaktionsbedingungen so zu wählen, dass die intramolekular ablaufende Ringschlussreaktion gegenüber intermolekularen

Reaktionen des gleichen Reaktionstyps, die zur Bildung von Dimeren oder ähnlichem führen würde, begünstigt ist. Zu diesem Zwecke kann man z.B., wenn es erforderlich ist, in grösserer Verdünnung arbeiten.

Die Aktivierung mit Hilfe der Base kann bei der gleichen oder einer anderen Temperatur erfolgen als die eigentliche Ringschlussreaktion.

Insbesondere bei Verwendung einer sehr starken Base, z.B. Lithium-diisopropylamid, erfolgt die Aktivierung mit der Base ("Metallierung") bei tieferer Temperatur (z.B. -80°C) als der Ringschluss. In diesem Fall kann man z.B. das Reaktionsgefäss nach erfolgter Metallierung sich langsam aufwärmen lassen.

Bei der Wahl des Lösungsmittels muss auch die Art der zu verwendenden Base berücksichtigt werden. Reaktionen unter Verwendung von Alkalimetallhydroxiden werden vorzugsweise in Dimethylsulfoxid oder in Alkoholen, wie Niederalkanolen, z.B. wasserfreiem Ethanol bei Siedetemperatur, Reaktionen unter Verwendung von Alkoholaten werden vorzugsweise in Ethern, insbesondere cyclischen Ethern, durchgeführt, bei Verwendung von Natrium-tert.butylat z.B. in Dioxan bei Raumtemperatur.
Ringschlussreaktionen mit z.B. 2-Oxo-pyrrolidin-natrium werden unter anderem in einem Gemisch aus einem cyclischen Ether und einem aromatischen Kohlenwasserstoff, z.B. einem Dioxan-Toluol-Gemisch, bei einer Temperatur zwischen Raumtemperatur und 60°C durchgeführt.

Die erfindungsgemässe Zyklisierung mit Hilfe eines Ionenaustauschers nimmt man insbesondere in einem Alkohol, z.B. Niederalkanol, z.B. Aethanol, bei Raumtemperatur vor.

Die Ausgangsstoffe der Formel II können nach an sich bekannten Methoden hergestellt werden, z.B. durch Acylierung der freien Aminogruppe eines Peptidamids der Formel X,

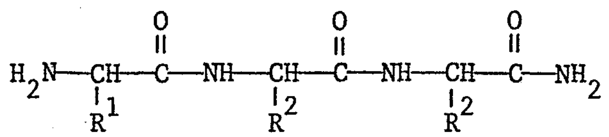

(X)

worin die Substituenten die obengenannten Bedeutungen haben, mit der Massgabe, dass darin vorhandene freie funktionelle Gruppen, wenn erwünscht, in geschützter Form vorliegen, mit einem Säurechlorid der Formel XI,

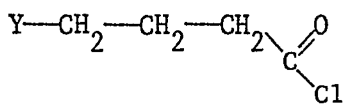

(XI)

worin Y die obengenannte Bedeutung hat, z.B. Y für Chlor steht, und nachfolgende Abspaltung der Schutzgruppen.

Verfahren b) (intramolekulare Zyklisierung einer Verbindung der Formel III):

Aktivierte Carbonsäurederivate einer Verbindung der Formel III sind in erster Linie reaktionsfähige aktivierte Ester oder reaktionsfähige Anhydride, ferner reaktionsfähige cyclische Amide; dabei können reaktionsfähige Derivate von Säuren der Formel III auch in situ gebildet werden.

Aktivierte Ester von Säuren sind insbesondere am Verknüpfungskohlenstoffatom des veresternden Restes ungesättigte Ester, z.B. vom Vinylester-Typ, wie eigentliche Vinylester (die man z.B. durch Umesterung eines entsprechenden Esters mit Vinylacetat erhalten kann; Methode des aktivierten Vinylesters), Carbamoylvinylester (die man z.B. durch

Behandeln der entsprechenden Säure mit einem Isoxazoliumreagens erhalten kann; 1,2-Oxazolium- oder Woodward-Methode), oder 1-Niederalkoxyvinylester (die man z.B. durch Behandeln der entsprechenden Säure mit einem Niederalkoxyacetylen erhalten kann; Ethoxyacetylen-Methode), oder Ester vom Amidinotyp, wie N,N'-disubstituierte Amidinoester (die man z.B. durch Behandeln der entsprechenden Säure mit einem geeigneten N,N'-disubstituierten Carbodiimid, z.B. N,N'-Dicyclohexylcarbodiimid, erhalten kann; Carbodiimid-Methode), oder N,N-disubstituierte Amidinoester (die man z.B. durch Behandeln der entsprechenden Säure mit einem N,N-disubstituierten Cyanamid erhalten kann; Cyanamid-Methode), geeignete Arylester, insbesondere durch Elektronenanziehende Substituenten geeignet substituierte Phenylester (die man z.B. durch Behandeln der entsprechenden Säure mit einem geeignet substituierten Phenol, z.B. 4-Nitrophenol, 4-Methylsulfonyl-phenol, 2,4,5-Trichlorphenol, 2,3,4,5,6-Pentachlor-phenol oder 4-Phenyldiazophenol, in Gegenwart eines Kondensationsmittels, wie N,N'-Dicyclohexyl-carbodiimid, erhalten kann; Methode der aktivierten Arylester), Cyanmethylester (die man z.B. durch Behandeln der entsprechenden Säure mit Chloracetonitril in Gegenwart einer Base erhalten kann; Cyanmethylester-Methode), Thioester, insbesondere gegebenenfalls, z.B. durch Nitro, substituierte Phenyl-thioester (die man z.B. durch Behandeln der entsprechenden Säure mit gegebenenfalls, z.B. durch Nitro, substituierten Thiophenolen, u.a. mit Hilfe der Anhydrid- oder Carbodiimid-Methode, erhalten kann; Methode der aktivierten Thiolester), oder Amino- oder Amidoester (die man z.B. durch Behandeln der entsprechenden Säure mit einer N-Hydroxy-amino- bzw. N-Hydroxy-amido-Verbindung, z.B. N-Hydroxy-succinimid, N-Hydroxy-piperidin, N-Hydroxyphthalimid oder 1-Hydroxy-benztriazol, z.B. nach der Anhydrid- oder Carbodiimid-Methode, erhalten kann; Methode der aktivierten N-Hydroxyester) oder Silylester (die man z.B. durch Behandeln der entsprechenden Säure mit einem Silylierungsmittel, z.B. Hexamethyldisilazan, erhalten kann ).

Anhydride von Säuren der Formel III können symmetrische oder vorzugsweise gemischte Anhydride dieser Säuren sein, so z.B. Anhydride mit anorganischen Säuren, wie Säurehalogenide, insbesondere Säurechloride (die man z.B. durch Behandeln der entsprechenden Säure mit Thionylchlorid, Phosphorpentachlorid oder Oxalylchlorid erhalten kann; Säurechloridmethode), Azide (die man z.B. aus einem entsprechenden Säureester über das entsprechende Hydrazid und dessen Behandlung mit salpetriger Säure erhalten kann; Azidmethode), Anhydride mit Kohlensäurehalbderivaten, wie mit entsprechenden Estern, z.B. Kohlensäureniederalkylhalbestern (die man z.B. durch Behandeln der entsprechenden Säure mit Halogen-, wie Chlorameisensäure-niederalkylestern oder mit einem 1-Niederalkoxycarbonyl-2-niederalkoxy-1,2-dihydro-chinolin, z.B. 1-Niederalkoxycarbonyl-2-ethoxy-1,2-dihydrochinolin, erhalten kann; Methode der gemischten O-Alkyl-kohlensäureanhydride), oder Anhydride mit dihalogenierter, insbesondere dichlorierter Phosphorsäure (die man z.B. durch Behandeln der entsprechenden Säure mit Phosphoroxychlorid erhalten kann; Phosphoroxychloridmethode), oder Anhydride mit organischen Säuren, wie gemischte Anhydride mit organischen Carbonsäuren (die man z.B. durch Behandeln der entsprechenden Säure mit einem gegebenenfalls substituierten Niederalkan- oder Phenylalkancarbonsäurehalogenid, z.B. Phenylessigsäure-, Pivalinsäure- oder Trifluoressigsäurechlorid erhalten kann; Methode der gemischten Carbonsäureanhydride) oder mit organischen Sulfonsäuren (die man z.B. durch Behandeln eines Salzes, wie eines Alkalimetallsalzes, der entsprechenden Säure, mit einem geeigneten organischen Sulfonsäurehalogenid, wie Niederalkan- oder Aryl-, z.B. Methan- oder p-Toluolsulfonsäurechlorid, erhalten kann; Methode der gemischten Sulfonsäureanhydride), sowie symmetrische Anhydride (die man z.B. durch Kondensation der entsprechenden Säure in Gegenwart eines Carbodiimids oder von 1-Diethylaminopropin erhalten kann; Methode der symmetrischen Anhydride).

Geeignete cyclische Amide sind insbesondere Amide mit fünfgliedrigen Diazacyclen aromatischen Charakters, wie Amide mit Imidazolen, z.B.

Imidazol (die man z.B. durch Behandeln der entsprechenden Säure mit N,N'-Carbonyldiimidazol; Imidazolid-Methode), oder Pyrazolen, z.B. 3,5-Dimethyl-pyrazol (die man z.B. über das Säurehydrazid durch Behandeln mit Acetylaceton erhalten kann; Pyrazolid-Methode).

Die an der Reaktion teilnehmende Aminogruppe in einem Ausgangsmaterial der Formel III liegt bevorzugterweise in freier Form vor, insbesondere wenn die damit reagierende Carboxygruppe in reaktionsfähiger Form vorliegt, sie kann aber auch selbst derivatisiert sein, d.h. z.B. durch Reaktion mit einem Phosphit, wie Diäthylchlorphosphit, 1,2-Phenylen-chlorphosphit, Ethyl-dichlor-phosphit, Ethylen-chlor-phosphit oder Tetraethylpyrophosphit, aktiviert worden sein.

Schutzgruppen von gegebenenfalls vorhandenen funktionellen Gruppen sind z.B. die obengenannten Schutzgruppen.

Die Ringschlussreaktion kann in an sich bekannter Weise durchgeführt werden, wobei die Reaktionsbedingungen in erster Linie davon abhängen, ob und wie die an der Reaktion teilnehmende Carboxylgruppe aktiviert ist, üblicherweise in Gegenwart eines geeigneten Lösungs- oder Verdünnungsmittels oder eines Gemisches von solchen, und, falls notwendig, in Gegenwart eines Kondensationsmittels, das z.B., wenn die an der Reaktion teilnehmende Carboxylgruppe als Anhydrid vorliegt, auch ein Säure-bindendes Mittel sein kann, unter Kühlen oder Erwärmen, z.B. in einem Temperaturbereich von etwa -30°C bis etwa +200°C, in einem geschlossenen Reaktionsgefäss und/oder in der Atmosphäre eines Inertgases, z.B. Stickstoff.

Generell sind die Reaktionsbedingungen so zu wählen, dass die intramolekular ablaufende Ringschlussreaktion gegenüber intermolekularen Reaktionen des gleichen Reaktionstyps, die zur Bildung von Dimeren oder ähnlichem führen würde, begünstigt ist. Zu diesem Zwecke kann man z.B., wenn es erforderlich ist, in grösserer Verdünnung arbeiten.

Die Reaktion kann z.B. durchgeführt werden, indem man eine Verbindung der Formel III, worin $R^3$ eine unaktivierte Carboxylgruppe bedeutet, und die übrigen gegebenenfalls in geschützter Form vorliegenden Reste die obengenannten Bedeutungen haben, gegebenenfalls in Gegenwart eines wasserentziehenden Mittels, z.B. eines N,N'-disubstituierten Carbodiimids, wie Dicyclohexylcarbodiimid, und gegebenenfalls zusätzlich in Gegenwart eines N-Hydroxy-amins oder N-Hydroxy-amids, z.B. N-Hydroxy-succinimid, in einem wasserfreien inerten Lösungsmittel erwärmt, z.B. auf 50°C-180°C.

Verbindungen der Formel III, worin $R^3$ eine aktivierte Carboxylgruppe bedeutet, reagieren in einem wasserfreien Lösungsmittel schon bei tieferen Temperaturen, z.B. -20° bis +50°C, unter Ausbildung des 2-Oxo-pyrrolidin-Ringes. Vorzugsweise geht man von solchen aktivierten Carbonsäureverbindungen der Formel III aus, worin die dem $\underline{C}$-$R^1$-Atom benachbarte Aminogruppe in geschützter Form vorliegt und setzt dann die Aminogruppe in situ frei, worauf die Zyklisierung eintritt.

Eine bevorzugte Ausführungsform des Verfahrens b) ist das Erhitzen einer Carbonsäure der Formel III in Hexamethyldisilazan.

Die Verbindungen der Formel III, worin die Carboxylgruppe $R^3$ in geschützter Form vorliegt, d.h. als Rest $R^3_a$, z.B. als 2-Trimethylsilylethylcarbonyl, können z.B. durch N-Alkylierung einer Verbindung der Formel X oder eines geeigneten Derivats davon, z.B. eines Azomethins oder des Alkalimetallsalzes eines Benzolsulfonamids, mit einer Verbindung der Formel XII,

$$R^3_a—CH_2—CH_2—CH_2—Y \qquad \text{(XII)}$$

worin $R^3_a$ eine geschützte Carboxylgruppe bedeutet und Y die obengenannte Bedeutung hat, hergestellt werden.

Verfahren c) (N-Alkylierung des 2-Oxo-pyrrolidins der Formel IV):

Eine reaktionsfähige Form einer Verbindung der Formel IV ist insbesondere eine Metallverbindung, in erster Linie eine Alkalimetallverbindung, z.B. eine Natrium-, Kalium- oder Lithiumverbindung derselben, die man z.B. durch Einwirkung einer entsprechenden, geeigneten Base, z.B. eines Alkalimetallhydroxids, z.B. Natriumhydroxid, eines Alkalimetallalkoholats, z.B. -niederalkanolats, wie -methylats, -ethylats oder tert.butylats, eines -hydrids, -amids, z.B. Natriumhydrid, Natriumamid oder Lithiumdiisopropylamid, oder einer Alkalimetall-Kohlenwasserstoffverbindung, z.B. Butyllithium, herstellen kann.

Die nucleophile Abgangsgruppe Y entspricht der bei Verfahren a) genannten Gruppe Y und ist vor allem Chlor oder Brom.

Die Reaktion wird vorzugsweise in einem wasserfreien oder, abhängig von der verwendeten Base, aprotischen Lösungsmittel bei Temperaturen zwischen etwa -80° C und +150° C und ähnlich wie bei Verfahren a) durchgeführt.
Die Ausgangsverbindungen der Formel V sind durch Acylierung einer Verbindung der Formel VII, worin q und r beide für 1 stehen, mit einem Acylierungsmittel der Formel XIII,

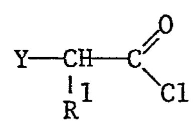

(XIII)

worin Y und $R^1$ die obengenannte Bedeutung haben, mit der Massgabe, dass im Rest $R^1$ vorhandene freie funktionelle Gruppen, wenn nötig, in geschützter Form vorliegen, und nachfolgende Abspaltung der Schutzgruppe(n) zugänglich.

Die Ausgangsverbindung der Formel IV ist bekannt und kann z.B. durch Zyklisierung von 4-Amino-buttersäure durch Erhitzen auf 200-250° C ohne Lösungsmittel und Kugelrohrdestillation erhalten werden.

Verfahren d) (Knüpfung einer Peptidbindung):

Reaktionsfähige Carbonsäurederivate einer Verbindung der Formel VI sind reaktionsfähige Ester, Anhydride oder Amide analog den bei Verfahren b) genannten.

Wie bei b) erwähnt können Derivate von Säuren der Formel VI auch in situ gebildet werden. So kann man z.B. N,N'-disubstituierte Amidinoester in situ bilden, indem man das Gemisch des Ausgangsmaterials der Formel VII und der Säure der Formel VI in Gegenwart eines geeigneten N,N'-disubstituierten Carbodiimids, z.B. N,N'-Dicyclohexylcarbodiimid, zur Reaktion bringt. Ferner kann man Amino- oder Amidoester von Säuren der Formel VI in Gegenwart des zu acylierenden Ausgangsmaterials der Formel VII bilden, indem man das Gemisch der entsprechenden Säure- und Amino-Ausgangsstoffe in Gegenwart eines N,N'-disubstituierten Carbodiimids, z.B. N,N'-Dicyclohexyl-carbodiimid, und eines N-Hydroxy-amins oder N-Hydroxy-amids, z.B. N-Hydroxysuccinimid, gegebenenfalls in Anwesenheit einer geeigneten Base, z.B. 4-Dimethylamino-pyridin, umsetzt.
Ein Derivat einer Verbindung der Formel VII, worin die an der Reaktion teilnehmende Aminogruppe in reaktionsfähiger Form vorliegt, kann z.B. durch Umsetzung mit einem Phosphit, z.B. einem der bei Verfahren b) genannten, hergestellt werden. Eine reaktionsfähige Form einer Verbindung der Formel VII ist z.B. auch ein Carbaminsäurehalogenid oder ein Isocyanat, wobei die an der Reaktion teilnehmende Aminogruppe in einer Verbindung der Formel VII an Halogencarbonyl, z.B. Chlorocarbonyl, gebunden ist, bzw. als Isocyanatogruppe vorliegt. Eine reaktionsfähige Form einer Verbindung der Formel VII, worin r für O steht, ist z.B. auch Harnstoff.

Beispielsweise erhält man beim Erhitzen äquimolarer Mengen Harnstoff und einer freien Säure der Formel VI die Verbindungen der Formel I in guter Ausbeute.

Die Acylierung einer Verbindung der Formel VII oder eines reaktionsfähigen Derivats davon mit einer Verbindung der Formel VI oder einem reaktionsfähigen Säurederivat davon kann in an sich bekannter Weise durchgeführt werden, wobei die Reaktionsbedingungen in erster Linie von der Art des verwendeten Acylierungsmittels abhängen, üblicherweise in Gegenwart eines geeigneten Lösungs- oder Verdünnungsmittels oder eines Gemisches von solchen, und, falls notwendig, in Gegenwart

eines Kondensationsmittels, das z.B. bei Verwendung eines Anhydrids als Acylierungsmittel gegebenenfalls auch ein Säure-bindendes Mittel sein kann, unter Kühlen oder Erwärmen, z.B. in einem Temperaturbereich von etwa -30°C bis etwa +150°C, in einem geschlossenen Reaktionsgefäss und/oder in der Atmosphäre eines Inertgases, z.B. Stickstoff.

Die Ausgangsstoffe der Formeln VI und VII sind bekannt oder können nach an sich bekannten Verfahren, z.B. analog den in dieser Anmeldung beschriebenen Verfahren, hergestellt werden.

Verfahren d) umfasst auch die Ausführungsform, wonach man zunächst eine Carbonsäure der Formel VI, worin n und p für 1 stehen, mit einer Verbindung der Formel VII, worin r für 0 steht, unter Bildung eines Ammoniumsalzes der Carbonsäure der Formel VI umsetzt, und das auf diese oder eine andere Art erhaltene Ammoniumsalz unter Bildung einer Verbindung der Formel I thermisch dehydratisiert, z.B. analog der im Britischen Patent 1 309 692 beschriebenen Art.

Verfahren e) (Amidherstellung aus einem Nitril): Die Ueberführung eines Nitrils in ein Amid kann durch partielle Hydrolyse oder auf dem Weg über Carbonsäureesterimid-Salze erfolgen. Die Bedingungen zur Hydrolyse einer Verbindung der Formel VIII müssen so gewählt werden, dass die Reaktion auf der Stufe des Amids angehalten werden kann und nicht die freie Carbonsäure gebildet wird. Zu diesem Zweck am generellsten geeignet ist die Hydrolyse mit Säuren, wobei man je nach den in einer Verbindung der Formel VIII vorhandenen Substituenten wählen kann zwischen 80%iger Schwefelsäure (unter Erwärmen), Polyphosphorsäure (bei 110-150°), Bromwasserstoff/Eisessig (Raumtemperatur), Ameisensäure (ohne Lösungsmittel), Chlorwasserstoffgas in etherischer Lösung gefolgt von Zugabe von Wasser oder wässeriger Salzsäure, oder Borhalogeniden/1 Aequivalent Wasser .

In einigen Fällen gelingt auch die alkalische Hydrolyse, jedoch ist die Methode von Radziszewski mit Wasserstoffperoxid in Gegenwart von Alkalien bei mässiger Temperatur meist erfolgreicher.

Bevorzugterweise verwendet man ein Ionenaustauscherharz, z.B. Lewatite® M 504 (vgl. Deutsche Offenlegungsschrift 2 507 576) oder Amberlite® IRA-400.

Die Carbonsäureesterimide werden z.B. durch säurekatalysierte Anlagerung von Alkoholen an die Nitrile der Formel VIII hergestellt. Aus den Esterimiden erhält man die Amide im Sinne einer Pinner-Spaltung. Statt mit einer starken Säure kann man auch mit Quecksilber(II)-nitrat katalysieren und anschliessend mit Natriumborhydrid reduzieren.

Nitrile der Formel VIII können beispielsweise durch eine Kolbe-Synthese aus den entsprechenden primären Halogeniden mit Cyanidionen im Sinne einer nucleophilen Substitution hergestellt werden. Bevorzugterweise setzt man z.B. ein primäres Halogenid mit Kupfercyanid in Dioxan um (vgl. Deutsche Offenlegungsschrift 2 507 576). Die primären Halogenide erhält man z.B. aus den primären Alkoholen mittels Thionylchlorid (vgl. Deutsche Offenlegungsschrift 2 507 576). Bevorzugterweise erhält man die Nitrile der Formel VIII, worin $R^2$ für Wasserstoff steht, durch Reaktion einer Verbindung der Formel VI, worin p für 1, n für 0 und $R^2$ für Wasserstoff steht, mit Aminoacetonitril, welches im Handel erhältlich ist, nach den für Verfahren d) beschriebenen Methoden.

Verfahren f) (Beckmann-Umlagerung):

Die Umlagerung von Verbindungen der Formel IX kann unter den bekannten Bedingungen der Beckmann-Umlagerung von Aldoximen durchgeführt werden, am besten mit Polyphosphorsäure oder Bortrifluorid.

Die Oxime der Formel IX sind nach an sich bekannten Methoden, z.B. durch Umsetzung der entsprechenden Aldehyde mit Hydroxylamin zugänglich.

Verfahren g) (Umwandlungen innerhalb der Definition der Endstoffe):

Die erfindungsgemäss erhältlichen Verbindungen der Formel (I) können in an sich bekannter Weise in andere Verbindungen der Formel (I) überführt werden.

In den Verbindungen der Formel (I), die mindestens eine freie Hydroxylgruppe aufweisen, kann diese nach an sich bekannten Methoden verethert oder verestert (acyliert) werden. Freies Carboxy kann verestert werden.

Geeignete Mittel zur Veretherung einer Hydroxylgruppe oder Veresterung einer Carboxylgruppe sind beispielsweise entsprechende Diazoverbindungen, wie gegebenenfalls substituierte Diazoniederalkane, z.B. Diazomethan, Diazoethan, Diazo-n-butan oder Diphenyldiazomethan. Diese Reagenzien werden in Gegenwart eines geeigneten inerten Lösungsmittels, wie eines aliphatischen, cycloaliphatischen oder aromatischen Kohlenwasserstoffs, wie Hexan, Cyclohexan, Benzol oder Toluol, eines halogenierten aliphatischen Kohlenwasserstoffs, z.B. Methylenchlorid, oder eines Ethers, wie eines Diniederalkylethers, z.B. Diethylether, oder eines cyclischen Ethers, z.B. Tetrahydrofuran oder Dioxan, oder eines Lösungsmittelgemisches, und, je nach Diazoreagens, unter Kühlen, bei Raumtemperatur oder unter leichtem Erwärmen, ferner, wenn notwendig, in einem geschlossenen Gefäss und/oder unter einer Inertgas-, z.B. Stickstoffatmosphäre, zur Anwendung gebracht.

Weitere geeignete Mittel zur Veretherung einer Hydroxylgruppe oder zur Veresterung einer Carboxylgruppe in einer Verbindung der Formel I sind Ester entsprechender Alkohole, in erster Linie solche mit starken anorganischen oder organischen Säuren, wie Mineralsäuren, z.B. Halogenwasserstoffsäuren, wie Chlorwasserstoff-, Bromwasserstoff- oder Jodwasserstoffsäure, ferner Schwefelsäure, oder Halogen-schwefelsäure, z.B. Fluorschwefelsäure, oder starken organischen Sulfonsäuren, wie gegebenenfalls, z.B. durch Halogen, wie Fluor, substituierten Niederalkansulfonsäuren, oder aromatischen Sulfonsäuren, wie z.B. gegebenenfalls durch Niederalkyl, wie Methyl, Halogen, wie Brom, und/oder Nitro substituierten Benzolsulfonsäuren, z.B. Methansulfon-, Trifluormethansulfon- oder p-Toluolsulfonsäure. Solche Ester sind u.a. Niederalkylhalogenide, Diniederalkylsulfate, wie Dimethylsulfat, ferner Fluorsulfonsäureester, wie -niederalkylester, z.B. Fluorsulfonsäuremethylester, oder gegebenenfalls Halogen-substituierte Methansulfonsäure-niederalkylester, z.B. Trifluormethansulfonsäuremethylester. Sie werden üblicherweise in Gegenwart eines inerten Lösungsmittels, wie eines gegebenenfalls halogenierten, wie chlorierten, aliphatischen, cycloaliphatischen oder aromatischen Kohlenwasserstoffs, z.B. Methylenchlorid, eines Ethers, wie Dioxan oder Tetrahydrofuran, oder eines Gemisches verwendet. Dabei wendet man vorzugsweise geeignete Kondensationsmittel, wie Alkalimetallcarbonate oder -hydrogencarbonate, z.B. Natrium- oder Kaliumcarbonat oder -hydrogencarbonat (üblicherweise zusammen mit einem Sulfat), oder organische Basen, wie üblicherweise sterisch gehinderte Triniederalkylamine, z.B. N,N-Diisopropyl-N-ethyl-amin (vorzugsweise zusammen mit Halogensulfonsäure-niederalkylestern oder gegebenenfalls halogensubstituierten Methansulfonsäureniederalkylestern) an, wobei unter Kühlen, bei Raumtemperatur oder unter Erwärmen, z.B. bei Temperaturen von etwa -20°C bis etwa 50°C und, wenn notwendig, in einem geschlossenen Gefäss und/oder in einer Inertgas-, z.B. Stickstoffatmosphäre, gearbeitet wird.

Durch Phasentransfer-Katalyse [siehe z.B. Dehmlow, Angewandte Chemie, 86, 187 (1974)] kann die oben beschriebene Veretherungsreaktion wesentlich beschleunigt werden. Als Phasentransfer- Katalysatoren können quartäre Phosphoniumsalze und insbesondere quartäre Ammoniumsalze, wie gegebenenfalls substituierte Tetraalkylammoniumhalogenide, z.B. Tetrabutylammoniumchlorid, -bromid oder -jodid, oder auch Benzyl-triethylammoniumchlorid, in katalytischen oder bis zu äquimolaren Mengen verwendet werden. Als organische Phase kann irgendeines der mit Wasser nicht mischbaren Lösungsmittel dienen, beispielsweise einer der gegebenenfalls halogenierten, wie chlorierten,niederaliphatischen, cycloaliphatischen oder aromatischen Kohlenwasserstoffe, wie Tri- oder Tetrachlorethylen, Tetrachlorethan, Tetrachlorkohlenstoff, Chlorbenzol, Toluol oder Xylol. Die als Kondensationsmittel geeigneten Alkalimetallcarbonate oder -hydrogencarbonate, z.B. Kalium- oder Natriumcarbonat oder -hydrogencarbonat, Alkalimetallphosphate, z.B. Kaliumphosphat, und Alkalimetallhydroxide, z.B. Natriumhydroxid, können bei basenempfindlichen Verbindungen der Reaktionsmischung titrimetrisch, z.B. mittels eines Titrierautomaten, zugesetzt werden, damit der pH-Wert während der Veretherung zwischen etwa 7 und etwa 8,5 bleibt.

Weitere Mittel zur Veretherung einer Hydroxylgruppe oder zur Veresterung einer Carboxylgruppe in einer Verbindung der Formel I sind entsprechende trisubstituierte Oxoniumsalze (sogenannte Meerweinsalze), oder disubstituierte Carbenium- oder Haloniumsalze, worin die Substituenten die verethernden Reste sind, beispielsweise Triniederalkyloxoniumsalze, sowie Diniederalkoxycarbenium- oder Diniederalkylhaloniumsalze, insbesondere die entsprechenden Salze mit komplexen, fluorhaltigen Säuren, wie die entsprechenden Tetrafluorborate, Hexafluorphosphate, Hexafluorantimonate, oder Hexachlorantimonate. Solche Reagentien sind z.B. Trimethyloxonium- oder Triethyloxonium-hexafluorantimonat, -hexachlorantimonat, -hexafluorphosphat oder -tetrafluorborat, Dimethoxycarbeniumhexafluorphosphat oder Dimethylbromoniumhexafluorantimonat. Man verwendet diese Veretherungsmittel vorzugsweise in einem inerten Lösungsmittel, wie einem Ether oder einem halogenierten Kohlenwasserstoff, z.B. Diethylether, Tetrahydrofuran oder Methylenchlorid,oder in einem Gemisch davon, wenn notwendig, in Gegenwart einer Base, wie einer organischen Base, z.B. eines, vorzugsweise sterisch gehinderten, Triniederalkylamins, z.B. N,N-Diisopropyl-N-ethyl-amin, und unter Kühlen, bei Raumtemperatur oder unter leichtem Erwärmen, z.B. bei etwa -20°C bis etwa 50°C, wenn notwendig, in einem geschlossenen Gefäss und/oder in einer Inertgas-, z.B. Stickstoffatmosphäre.

Weitere geeignete Veretherungsmittel sind schliesslich entsprechende 1-substituierte 3-Aryltriazenverbindungen, worin der Substituent den verethernden Rest, und Aryl vorzugsweise gegebenenfalls substituiertes Phenyl, z.B. Niederalkylphenyl, wie 4-Methylphenyl, bedeutet. Solche Triazenverbindungen sind 3-Aryl-1-niederalkyltriazene, z.B. 3-(4-Methylphenyl)-1-methyl-triazen, 3-(4-Methylphenyl)-1-ethyl-triazen oder 3-(4-Methylphenyl)-1-isopropyl-triazen. Diese Reagentien werden üblicherweise in Gegenwart von inerten Lösungsmitteln, wie gegebenenfalls halogenierten Kohlenwasserstoffen oder Ethern, z.B. Benzol, oder Lösungsmittelgemischen, und unter Kühlen, bei Raumtemperatur und vorzugsweise bei erhöhter Temperatur, z.B. bei etwa 20°C bis etwa 100°C, wenn notwendig in einem geschlossenen Gefäss und/oder in einer Inertgas-, z.B. Stickstoffatmosphäre, verwendet.

Die Umwandlung von freiem Carboxyl in einer Verbindung der Formel (I) in verestertes Carboxyl, kann weiterhin beispielsweise erfolgen, indem man eine Verbindung der Formel (I), worin andere, gegebenenfalls vorhandene funktionelle Gruppen gegebenenfalls in geschützter Form vorliegen, oder ein reaktionsfähiges funktionelles Carboxyderivat, inkl. ein Salz davon, mit einem entsprechenden Alkohol oder einem reaktionsfähigen funktionellen Derivat davon umsetzt.

Die Veresterung von freiem Carboxyl mit dem gewünschten Alkohol wird in Gegenwart eines geeigneten Kondensationsmittels durchgeführt. Uebliche Kondensationsmittel sind z.B. Carbodiimide, beispielsweise N,N'-Diethyl-, N,N'-Dipropyl-, N,N'-Dicyclohexyl- oder N-Ethyl-N'-(3-dimethylaminopropyl)-carbodiimid, geeignete Carbonylverbindungen, beispielsweise Carbonyldiimidazol, oder 1,2-Oxazoliumverbindungen, z.B. 2-Ethyl-5-phenyl-1,2-oxazolium-3'-sulfonat und 2-tert.-Butyl-5-methyl-isoxazolium-perchlorat, oder eine geeignete Acylaminoverbindung, z.B. 2-Ethoxy-1-ethoxycarbonyl-1,2-dihydrochinolin. Die Kondensationsreaktion wird vorzugsweise in einem wasserfreien Reaktionsmedium, vorzugsweise in Gegenwart eines Lösungs- oder

Verdünnungsmittels, z.B. Methylenchlorid, Dimethylformamid, Acetonitril oder Tetrahydrofuran und, wenn notwendig, unter Kühlen oder Erwärmen und/oder in einer Inertgasatmosphäre durchgeführt.

Geeignete reaktionsfähige funktionelle Derivate der zu veresternden Carboxylverbindungen der Formel (I) sind z.B. Anhydride, insbesondere gemischte Anhydride, und aktivierte Ester.

Gemischte Anhydride sind z.B. diejenigen mit anorganischen Säuren, wie Halogenwasserstoffsäuren, d.h. die entsprechenden Säurehalogenide, z.B. -chloride oder -bromide, ferner Stickstoffwasserstoffsäuren, d.h. die entsprechenden Säureazide, sowie phosphorhaltigen Säuren, z.B. Phosphorsäure, Diethylphosphorsäure oder phosphorige Säure, oder schwefelhaltigen Säuren, z.B. Schwefelsäure, oder Cyanwasserstoffsäure. Gemischte Anhydride sind weiter z.B. diejenigen mit organischen Carbonsäuren, wie mit gegebenenfalls, z.B. durch Halogen, wie Fluor oder Chlor, substituierten Niederalkancarbonsäuren, z.B. Pivalinsäure oder Trichloressigsäure, oder mit Halbestern, insbesondere Niederalkylhalbestern der Kohlensäure, wie Ethyl- oder Isobutylhalbestern der Kohlensäure, oder mit organischen, insbesondere aliphatischen oder aromatischen Sulfonsäuren, z.B. p-Toluolsulfonsäure.

Zur Reaktion mit einem Alkohol geeignete aktivierte Ester einer Verbindung der Formel I mit mindestens einer Carboxylgruppe sind z.B. Ester mit vinylogen Alkoholen (d.h. Enolen), wie vinylogen Niederalkenolen, oder Iminomethylesterhalogeniden, wie Dimethyliminomethylesterchlorid (hergestellt aus der Carbonsäure und Dimethylchlormethyliden-iminium-chlorid der Formel $[(CH_3)_2N=CHCl]^{\oplus}Cl^{\ominus}$), oder Arylester, wie Pentachlorphenyl-, 4-Nitrophenyl- oder 2,3-Dinitrophenylester, heteroaromatische Ester, wie Benztriazol-, z.B. 1-Benztriazolester, oder Diacyliminoester, wie Succinylimino- oder Phthalyliminoester.

Die Reaktion zwischen einem solchen Säurederivat der Formel I, wie einem Anhydrid, insbesondere einem Säurehalogenid, und einem Alkohol wird bevorzugt in Anwesenheit eines säurebindenden Mittels, beispielsweise einer organischen Base, wie eines organischen Amins, z.B. eines tertiären Amins, wie Triniederalkylamin, z.B. Trimethylamin, Triethylamin oder Ethyldiisopropylamin, oder N,N-Diniederalkyl-anilin, z.B. N,N-Dimethylanilin, oder eines cyclischen tertiären Amins, wie eines N-niederalkylierten Morpholins, wie N-Methylmorpholin, oder einer Base vom Pyridin-Typ, z.B. Pyridin, einer anorganischen Base, beispielsweise eines Alkalimetall- oder Erdalkalimetallhydroxids, -carbonats oder -hydrogencarbonats, z.B. Natrium-, Kalium- oder Calciumhydroxid, -carbonat oder -hydrogencarbonat, oder eines Oxirans, beispielsweise eines niederen 1,2-Alkylenoxids, wie Ethylenoxid oder Propylenoxid, durchgeführt.

Ein reaktionsfähiges funktionelles Derivat des veresternden Alkohols ist in erster Linie ein entsprechender Ester, vorzugsweise mit einer starken anorganischen oder organischen Säure und stellt insbesondere ein entsprechendes Halogenid, z.B. Chlorid, Bromid oder Jodid, oder eine entsprechende Niederalkyl- oder Aryl-, wie Methyl- oder 4-Methylphenylsulfonyloxyverbindung dar.

Ein solcher reaktionsfähiger Ester eines Alkohols kann mit der freien Carboxylverbindung der Formel (I) oder einem Salz, wie einem Alkalimetall- oder Ammoniumsalz, davon umgesetzt werden, wobei man bei Verwendung der freien Säure vorzugsweise in Gegenwart eines säurebindenden Mittels arbeitet.

Die obigen Veresterungsreaktionen werden in einem inerten, üblicherweise wasserfreien Lösungsmittel oder Lösungsmittelgemisch vorgenommen, beispielsweise in einem Carbonsäureamid, wie einem Formamid, z.B. Dimethylformamid, einem halogenierten Kohlenwasserstoff, z.B. Methylenchlorid, Tetrachlorkohlenstoff oder Chlorbenzol, einem Keton, z.B. Aceton, einem Ester, z.B. Essigsäureethylester, oder

einem Nitril, z.B.Acetonitril, oder Mischungen davon, wenn notwendig unter Kühlen oder Erwärmen, z.B. in einem Temperaturbereich von etwa -40°C bis etwa +100°C, vorzugsweise bei etwa -10°C bis etwa +40°C, und/oder in einer Inertgas-, z.B. Stickstoffatmosphäre.

Ferner kann das Säurederivat, wenn erwünscht, in situ gebildet werden. So erhält man z.B. ein gemischtes Anhydrid durch Behandeln der Carbonsäureverbindung mit entsprechend geschützten funktionellen Gruppen oder eines geeigneten Salzes davon, wie eines Ammoniumsalzes, z.B. mit einem organischen Amin, wie Piperidin oder 4-Methylmorpholin, oder eines Metall-, z.B. Alkalimetallsalzes mit einem geeigneten Säurederivat, wie dem entsprechenden Säurehalogenid einer gegebenenfalls substituierten Niederalkancarbonsäure, z.B. Trichloracetylchlorid, mit einem Halbester eines Kohlensäure-halbhalogenids, z.B. Chlorameisensäurehalbester oder -isobutylester, oder mit einem Halogenid einer Diniederalkylphosphorsäure, z.B. Diethylphosphorbromidat, und verwendet das so erhältliche gemischte Anhydrid ohne Isolierung.

Zur Veresterung (Acylierung) einer Hydroxygruppe in einer Verbindung der Formel I behandelt man das Ausgangsmaterial der Formel (I) mit einem, den gewünschten Acylrest einer organischen Carbonsäure einführenden Acylierungsmittel. Dabei verwendet man die entsprechende Carbonsäure oder ein reaktionsfähiges Derivat davon, insbesondere ein Anhydrid, inkl. ein gemischtes oder inneres Anhydrid einer solchen Säure. Gemischte Anhydride sind z.B. diejenigen mit Halogenwasserstoffsäuren, d.h. die entsprechenden Säurehalogenide, insbesondere -chloride, ferner mit Cyanwasserstoffsäure,oder dann diejenigen mit geeigneten Kohlensäurehalbderivaten, wie entsprechenden -halbestern (wie die z.B. mit einem Halogen-ameisensäure-niederalkyl, wie Chlorameisensäure-ethylester oder -isobutylester, gebildeten gemischten Anhydride) oder mit gegebenenfalls substituierten, z.B. Halogen, wie Chlor, enthaltenden Niederalkancarbonsäuren (wie die mit Pivalinsäurechlorid oder Trichloressigsäurechlorid gebildeten gemischten Anhydride). Innere Anhydride sind z.B. diejenigen von organischen Carbonsäuren, d.h. Ketene, wie Keten oder Diketen, oder diejenigen von Carbamin- oder Thiocarbaminsäuren, d.h. Isocyanate oder Isothiocyanate. Weitere reaktionsfähige, als Acylierungsmittel verwendbare Derivate von organischen Carbonsäuren sind aktivierte Ester, wie geeignet substituierte Niederalkyl-, z.B. Cyanmethylester, oder geeignet substituierte Phenyl-, z.B. Pentachlorphenyl- oder 4-Nitrophenylester. Die Veresterung kann, wenn notwendig, in Gegenwart von geeigneten Kondensationsmitteln, bei Verwendung von freien Carbonsäuren, z.B. in Gegenwart von Carbodiimidverbindungen, wie Dicyclohexylcarbodiimid, oder Carbonylverbindungen, wie Diimidazolylcarbonyl, und bei Verwendung von reaktionsfähigen Säurederivaten z.B. in Gegenwart von basischen Mitteln, wie Triniederalkylaminen, z.B. Triethylamin, oder heterocyclischen Basen, z.B. Pyridin, durchgeführt werden. Die Acylierungsreaktion kann in Abwesenheit oder in Gegenwart eines Lösungsmittels oder Lösungsmittelgemisches, unter Kühlen, bei Raumtemperatur oder unter Erwärmen, und, wenn notwendig, in einem geschlossenen Gefäss und/oder in einer Inertgas-, z.B. Stickstoffatmosphäre, durchgeführt werden. Geeignete Lösungsmittel sind z.B. gegebenenfalls substituierte, insbesondere gegebenenfalls chlorierte, aliphatische, cycloaliphatische oder aromatische Kohlenwasserstoffe, wie Benzol oder Toluol, wobei man geeignete Veresterungsreagentien, wie Essigsäureanhydrid, auch als Verdünnungsmittel verwenden kann.

Eine durch eine organische Sulfonsäure, z.B. Niederalkansulfonsäure, wie Methansulfonsäure, oder eine aromatische Sulfonsäure, wie p-Toluolsulfonsäure, veresterte Hydroxygruppe kann man vorzugsweise durch Behandeln des Ausgangsmaterials der Formel (I) mit einem reaktionsfähigen Sulfonsäurederivat, wie einem entsprechenden Halogenid, z.B. Chlorid, wenn notwendig, in Gegenwart eines Säure-neutralisierenden basischen Mittels, z.B. einer anorganischen oder organischen Base, z.B. in analoger Weise wie die entsprechenden Ester mit organischen Carbonsäuren, bilden.

Die zur Ausführung der vorstehend beschriebenen Verfahren a) bis g) benötigten Ausgangsstoffe sind bekannt oder können nach an sich bekannten Verfahren, z.B. nach oder analog den in dieser Anmeldung beschriebenen hergestellt werden.

Funktionelle Gruppen in Ausgangsstoffen können mit den gleichen Schutzgruppen geschützt werden, wie sie für entsprechende funktionelle Gruppen in den Endstoffen der Formel I in dieser Anmeldung genannt sind. Auch die Einführung und Abspaltung dieser Schutzgruppen kann in der Weise geschehen, wie sie in den zitierten Literaturstellen geschildert ist.

In einer erhaltenen Verbindung der Formel (I), worin eine oder mehrere funktionelle Gruppen geschützt sind, können diese, z.B. geschützte Carboxyl-, und/oder Hydroxygruppen, in an sich bekannter Weise, mittels Solvolyse, insbesondere Hydrolyse, Alkoholyse oder Acidolyse, oder in einigen Fällen auch mittels vorsichtiger Reduktion, gegebenenfalls stufenweise oder gleichzeitig freigesetzt werden. Silylschutzgruppen werden vorteilhafterweise mit Fluoriden, z.B. Tetraethylammoniumfluorid, abgespalten.

Salze von Verbindungen der Formel (I) mit salzbildenden Gruppen können in an sich bekannter Weise hergestellt werden. So kann man Salze von Verbindungen der Formel (I) mit sauren Gruppen, z.B. durch Behandeln mit Metallverbindungen, wie Alkalimetallsalzen von geeigneten organischen Carbonsäuren, z.B. dem Natriumsalz der α-Ethyl-capronsäure, oder mit anorganischen Alkali- oder Erdalkalimetallsalzen, z.B. Natriumhydrogencarbonat, oder mit Ammoniak oder einem geeigneten organischen Amin bilden, wobei man vorzugsweise stöchiometrische Mengen oder nur einen kleinen Ueberschuss des salzbildenden Mittels verwendet.

Salze können in üblicher Weise in die freien Verbindungen übergeführt werden, z.B. durch Behandeln mit geeigneten Säuren.

Gemische von Isomeren können in an sich bekannter Weise, z.B. durch fraktionierte Kristallisation, Chromatographie etc. in die einzelnen Isomeren aufgetrennt werden.

Racemate können in an sich bekannter Weise, z.B. nach Ueberführung der optischen Antipoden in Diastereomere, beispielsweise durch Umsetzung mit optisch aktiven Säuren oder Basen, gespalten werden.

Die Erfindung betrifft auch diejenigen Ausführungsformen des Verfahrens, bei denen man von einer auf irgendeiner Stufe als Zwischenprodukt erhältlichen Verbindung ausgeht und die fehlenden Schritte durchführt oder man das Verfahren auf irgendeiner Stufe abbricht oder man eine nach dem erfindungsgemässen Verfahren erhältliche Verbindung unter den Verfahrensbedingungen erzeugt und in situ weiterverarbeitet.

Neue Ausgangsstoffe und/oder Zwischenprodukte sowie Verfahren zu ihrer Herstellung sind ebenfalls Gegenstand der vorliegenden Erfindung. Vorzugsweise werden solche Ausgangsstoffe verwendet und die Reaktionsbedingungen so gewählt, dass man zu den in dieser Anmeldung als besonders bevorzugt aufgeführten Verbindungen gelangt.

Die pharmakologisch verwendbaren Verbindungen der vorliegenden Erfindung können z.B. zur Herstellung von pharmazeutischen Präparaten verwendet werden, welche eine wirksame Menge der Aktivsubstanz zusammen oder im Gemisch mit anorganischen oder organischen, festen oder flüssigen, pharmazeutisch verwendbaren Trägerstoffen enthalten.

Bei den erfindungsgemässen pharmazeutischen Präparaten handelt es sich um solche zur enteralen, wie oralen oder rektalen sowie parenteralen, wie intraperitonealen, intramuskulären oder intravenösen, Verabreichung an Warmblüter, welche den pharmakologischen Wirkstoff allein oder zusammen mit einem pharmazeutisch anwendbaren Trägermaterial enthalten.

Die Dosierung des Wirkstoffes hängt von der Warmblüter-Spezies, dem Körpergewicht, Alter und dem individuellen Zustand, der zu behandelnden Krankheit sowie von der Applikationsweise ab.

Die Dosierung liegt zwischen etwa 0,1 und etwa 100 mg/kg täglich, vorzugsweise zwischen etwa 0,1 und etwa 40 mg/kg täglich, z.B. bei etwa 10 mg/kg täglich.

Die an Warmblüter, z.B. Menschen, von etwa 70 kg Körpergewicht zu verabreichenden Dosen liegen zwischen etwa 10 mg und etwa 10 g, vorzugsweise zwischen etwa 100 mg und 2 g, z.B. bei 600 mg, pro Warmblüter und Tag, verteilt auf bis zu etwa 12, vorzugsweise 2 bis 4, z.B. 3, Einzeldosen, die z.B. gleich gross sein können. Die Dosierung bei enteraler und parenteraler Applikation ist im wesentlichen gleich. Die Abhängigkeit der Dosierung vom Körpergewicht ist nicht linear, sondern eher schwach ausgeprägt, so dass man die obengenannte Dosierung auch bei Warmblütern mit geringerem oder höherem Körpergewicht als 70 kg, z.B. 35 kg bzw. 100 kg, anwenden kann. Ueblicherweise jedoch erhalten Kinder die halbe Erwachsenendosis.

Die neuen pharmazeutischen Präparate enthalten eine signifikante Menge, insbesondere von etwa 1 % bis etwa 99 %, hauptsächlich von etwa 10 % bis etwa 95 %, vorzugsweise von etwa 20 % bis etwa 90 % des Wirkstoffes. Erfindungsgemässe pharmazeutische Präparate können z.B. in Dosiseinheitsform, wie Dragées, Tabletten, Kapseln, Suppositorien oder Ampullen, vorliegen.

Die pharmazeutischen Präparate der vorliegenden Erfindung werden in an sich bekannter Weise, z.B. mittels konventioneller Misch-, Granulier-, Dragier-, Lösungs- oder Lyophilisierungsverfahren, hergestellt. Pharmazeutische Präparate zur oralen Anwendung können erhalten werden, indem man den Wirkstoff mit festen Trägerstoffen kombiniert, ein erhaltenes Gemisch gegebenenfalls granuliert und das Gemisch bzw. Granulat, wenn erwünscht oder notwendig nach Zugabe von geeigneten Hilfsstoffen, zu Tabletten oder Dragée-Kernen verarbeitet. Dabei kann man sie auch in Kunststoffträger einbauen, die die Wirkstoffe dosiert abgeben oder diffundieren lassen.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose, Hydroxypropyl-methylcellulose, Natriumcarboxymethylcellulose und/oder Polyvinylpyrrolidon, und/oder, wenn erwünscht, Sprengmittel, wie die obengenannten Stärken, ferner Carboxymethylstärke, quervernetztes Poylvinylpyyrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls magensaftresistenten Ueberzügen versehen, wobei man u.a. konzentrierte Zuckerlösungen, welche gegebenenfalls arabischen Gummi, Talk, Polyvinylpyrrolidon, Polyäthylenglykol und/oder Titandioxid enthalten, Lacklösungen in geeigneten organischen Lösungsmitteln oder Lösungsmittelgemischen oder, zur Herstellung von magensaftresistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Die Herstellung der Injektionspräparate erfolgt in üblicher Weise unter antimikrobiellen Bedingungen, ebenso das Abfüllen in Ampullen oder Vials sowie das Verschliessen der Behälter.

Zur Herstellung der Injektionspräparate verwendet man vorzugsweise Lösungen des Wirkstoffes, daneben auch Suspensionen, und zwar insbesondere isotonische wässerige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder

Hilfsstoffe, z.B. Konservier-, Stabilisier-, Netz und/oder Emulgiermittel, Löslichkeitsvermittler, Salze zur Regulierung des osmotischen Druckes und/oder Puffer enthalten und werden in an sich bekannter Weise, z.B. mittels konventioneller Lösungs- oder Lyophilisierungsverfahren, hergestellt. Die genannten Lösungen oder Suspensionen können viskositätserhöhende Stoffe, wie Natrium-Carboxymethylcellulose, Carboxymethylcellulose, Dextran, Polyvinylpyrrolidon oder Gelatine, enthalten.

Die folgenden Beispiele dienen zur Illustration der Erfindung. Temperaturen werden in Celsiusgraden angegeben. $R_f$ - Werte werden, wenn nicht anders angegeben, auf Kieselgeldünnschichtplatten (Merck, Darmstadt, Deutschland) ermittelt.

Beispiel 1: Bei 15° versetzt man 15,64 g (0,4 Mol) 2-(2-Oxo-1-pyrrolidinyl)-essigsäure-pentachloro-phenyl-ester,6,68 g (0,4 Mol) Glycyl-glycinamid-hydrochlorid in 108 ml Dimethylformamid (DMF) auf einmal mit 6 ml Triethylamin, worauf die Temperatur spontan bis 25° steigt. Danach erwärmt man innerhalb von 30 Minuten unter Rühren auf 60° und rührt noch 1 Stunde bei 60-65°. Man kühlt auf 20° ab, versetzt mit 200 ml Ether, saugt die festen Bestandteile ab und wäscht mit Ether nach. Zum Entfernen von begleitendem Triethylammoniumchlorid wird der erhaltene Festkörper in 40 ml Methanol und 40 ml Ethanol gerührt, abgesaugt und mit Ethanol und Ethylether gewaschen. Den Rückstand löst man in 320 ml Methanol in der Hitze, entfärbt die Lösung mit Tierkohle, filtriert und wäscht mit 100 ml heissem Methanol nach. Das Filtrat wird am Rotationsverdampfer, zum Schluss bei 60°/13 Torr, auf 70 ml eingeengt und im Eisbad gekühlt. Die nach dem Absaugen und Waschen mit absolutem Ethanol erhaltenen Kristalle von N-[(2-Oxo-1-pyrrolidinyl)-acetyl]-glycyl-glycinamid schmelzen bei 169-170°, werden bei 171° wieder fest und schmelzen erneut bei 189-190°.

| | | | | | | | | |
|---|---|---|---|---|---|---|---|---|
| $C_{10}H_{16}N_4O_4$ (256,26) | Ber. | C | 46,87 | H | 6,29 | N | 21,86 |
| | Gef. | C | 46,71 | H | 6,28 | N | 21,56 |

Den als Ausgangsprodukt verwendeten aktivierten Ester stellt man folgendermassen her:

Zu 14,3 g (0,1 Mol) 2-(2-Oxo-1-pyrrolidinyl)-essigsäure und 29,3 g (0,11 Mol) Pentachlorophenol in 100 ml Methylenchlorid gibt man unter Rühren bei Raumtemperatur 22,7 g (0,11 Mol) festes Dicyclohexylcarbodiimid in 5 Portionen, wobei die anfangs dünnflüssige Suspension immer zähflüssiger wird und sich etwas erwärmt. Nach vollendeter Zugabe des Dicyclohexylcarbodiimids kocht man 6 Stunden unter Rückfluss, kühlt dann auf 10°, saugt den gebildeten Dicyclohexylharnstoff ab und wäscht dreimal mit Methylenchlorid. Das Filtrat wird bis zur Zähflüssigkeit eingeengt und mit 100 ml Diethylether und 100 ml Pentan versetzt. Das auskristallisierte Produkt mit Schmelzpunkt 125-131° wird in 1,5 l Diethylether gelöst, eingeengt und mit Petrolether versetzt. Nach dem Absaugen der auskristallisierten Substanz, Waschen und Trocknen, erhält man 2-(2-Oxo-1-pyrrolidinyl)-essigsäure-pentachlorophenylester mit Schmelzpunkt 137-138°.

Beispiel 2: In analoger Weise, wie in dieser Anmeldung beschrieben ist, erhält man N-[2-(2-Oxo-1-pyrrolidinyl)-butyryl]-glycyl-glycinamid und N-[2-(2-Oxo-1-pyrrolidinyl)-propionyl]-glycyl-glycinamid.

Beispiel 3: Rezept für eine Tablette:

| | | |
|---|---|---|
| N-[(2-Oxo-1-pyrrolidinyl)-acetyl]-glycyl-glycinamid | 100 | mg |
| Stärke | 15 | mg |
| Polyvinylpyrrolidon | 2 | mg |
| Talkum | 6 | mg |
| Magnesiumstearat | 1 | mg |

Beispiel 4: Herstellung von 1000 Tabletten, die je 100 mg N-[(2-Oxo-1-pyrrolidinyl)-acetyl]-glycyl-glycinamid enthalten:

Zusammensetzung

| | | |
|---|---|---|
| N-[(2-Oxo-1-pyrrolidinyl)-acetyl]-glycyl-glycinamid | 100 | g |
| Lactose | 100 | g |
| Kartoffelstärke | 70 | g |
| Gelatine | 1,6 | g |
| Talk | 12 | g |
| Magnesiumstearat | 2 | g |
| Siliciumdioxid (hochdispers) | 4 | g |
| Ethanol | q.s. | |

Der Wirkstoff wird mit der Lactose und 60 g Kartoffelstärke vermischt, die Mischung mit einer alkoholischen Lösung der Gelatine befeuchtet und durch ein Sieb granuliert. Nach dem Trocknen mischt man den Rest der Kartoffelstärke, den Talk, das Magnesiumstearat und das hochdisperse Siliciumdioxid zu und presst das Gemisch zu Tabletten von je 289,6 mg Gewicht und dem oben angegebenen Wirkstoffgehalt, die gewünschtenfalls mit Teilkerben zur feineren Anpassung der Dosierung versehen sein können.

Beispiel 5: In 100 ml Dimethylformamid werden 12,6 g (30 mMol) (D,L)-2-(2-Oxo-1-pyrrolidinyl)-buttersäure-pentachlorphenylester und 5,0 g Glycyl-glycinamid-hydrochlorid eingerührt. Unter Stickstoffatmosphäre wird bei 40°C 18 Stunden gerührt. Die entstandene leicht braune Suspension wird auf 10°C abgekühlt und filtriert. Auf dem Filter verbleiben 3,3 g Triethylamin-hydrochlorid, Smp. 254-6°. Das Filtrat wird bei 80°/13 Torr eingedampft. Der Rückstand, 20 g braunes Oel, wird an 300 g Silicagel (Merck, Kieselgel 60,70-230 mesh) chromatographiert. Die Fraktionen werden mit Ethanol eluiert. Die ersten Fraktionen enthalten Pentachlorphenol. Die folgenden Fraktionen werden vereinigt,vom Ethanol befreit und aus Methanol-Ether kristallisiert, worauf man (D,L)-N-[2-(2-Oxo-1-pyrrolidinyl)-butyryl]-glycyl-glycinamid mit Schmelzpunkt 146-149° erhält.

| | | | | | | | | |
|---|---|---|---|---|---|---|---|---|
| $C_{12}H_{20}N_4O_4$ | (284,32) | Ber. | C | 50,7 | H | 7,1 | N | 19,7 |
| | | Gef. | C | 50,8 | H | 7,2 | N | 19,5 |

Den Ausgangsstoff erhält man folgendermassen:
Zu einer Lösung von 29,3 g (0,11 Mol) Pentachlorphenol und 17,1 g (0,10 Mol) (D,L)-2-(2-Oxo-1-pyrrolidinyl)-buttersäure in 370 ml Tetrahydrofuran werden bei 10°C innerhalb von 30 Minuten 22,7 g (0,11 Mol) N,N-Dicyclohexylcarbodiimid, gelöst in 30 ml Tetrahydrofuran zugetropft. Nach 58 Stunden Rühren bei 40° wird die weisse Suspension abgesaugt. Das klare, leicht rosa gefärbte Filtrat wird bis zur Trockne eingedampft und aus Ether-Petrolether bei 0°C kristallisiert. Nach Umkristallisation aus Aceton-Hexan erhält man 33,1 g (D,L)-2-(2-Oxo-1-pyrrolidinyl)-buttersäure-pentachlorphenylester mit Schmelzpunkt 107-109°.

| | | | | | |
|---|---|---|---|---|---|
| $C_{14}H_{12}Cl_5NO_3$ | (419,52) | Ber. | C 40,08 | H 2,88 | N 3,34 |
| | | Gef. | C 40,2 | H 3,0 | N 3,6 |

Beispiel 6: 2,74 g (0,01 Mol) N-(3-Carboxy-propyl)-glycyl-glycyl-glycinamid werden in 20 ml Hexamethyldisilazan suspendiert und unter Rühren zum Sieden erhitzt. Nach kurzer Zeit bildet sich eine klare Lösung. Man erhitzt während 24 Stunden unter Rückfluss und dampft dann das rohe Reaktionsgemisch im Hochvakuum ein. Der Rückstand wird in 30 ml Methanol gelöst und bei Raumtemperatur gerührt, wobei nach kurzer Zeit das in Beispiel 1 beschriebene N-[(2-Oxo-1-pyrrolidinyl)-acetyl]-glycyl-glycinamid ausfällt. Dieses wird abgenutscht und mit wenig kaltem Methanol nachgewaschen.

Das Ausgangsmaterial wird wie folgt hergestellt.

10,3 g (0,10 Mol) 3-Amino-buttersäure, 28 g (0,20 Mol) Kaliumcarbonat und 12,5 g (0,05 Mol) N-(Brom-acetyl)-glycyl-glycinamid werden in 200 ml Ethanol während 8 Stunden bei 50° gerührt. Hierauf wird mit 2N Salzsäure vorsichtig neutralisiert und dann im Wasserstrahlvakuum eingedampft. Der Rückstand wird mit 250 ml Isopropanol versetzt und zum Kochen erwärmt. Die unlöslichen Salze werden abgenutscht und das Filtrat am Rotationsverdampfer bis zur beginnenden Kristallisation eingeengt, worauf man N-(3-Carboxy-propyl)-glycyl-glycyl-glycinamid erhält, das direkt weiterverarbeitet wird.

Beispiel 7: 2,92 g (0,01 Mol) N-(4-Chlor-butyryl)-glycyl-glycyl-glycinamid und 1,5 g (0,011 Mol) Kaliumcarbonat werden unter Rühren in 100 ml Ethanol während 5 Stunden zum Sieden erhitzt. Hierauf dampft man im Rotationsverdampfer ein, digeriert den Rückstand in 200 ml kochendem Isopropanol, filtriert von den unlöslichen anorganischen Salzen ab und engt im Wasserstrahlvakuum bis zur beginnenden Kristallisation ein, wobei das im Beispiel 1 beschriebene N-[(2-Oxo-1-pyrrolidinyl)-acetyl]-glycyl-glycinamid ausfällt, das abgenutscht und mit wenig Isopropanol nachgewaschen wird.

Das Ausgangsmaterial wird wie folgt hergestellt:

Zu einer Mischung von 2,1 g (0,01 Mol) Glycyl-glycyl-glycinamid-hydrochlorid und 2,0 g (0,02 Mol) Triethylamin in 50 ml Dimethylformamid lässt man unter Rühren bei einer Reaktionstemperatur von 5° 1,5 g (0,011 Mol) 4-Chlor-buttersäurechlorid zutropfen. Nach erfolgter Zugabe lässt man noch 1 Stunde bei 5-20° ausreagieren. Dann wird das Dimethylformamid im Hochvakuum abdestilliert. Der Rückstand wird mit 200 ml Ethanol aufgeschlämmt und das unlösliche N-(4-Chlor-butyryl)-glycyl-glycinamid abgenutscht.

Beispiel 8: Zu einer Lösung von 1,70 g (0,02 Mol) 2-Oxo-pyrrolidin in 30 ml Dimethylformamid gibt man unter Rühren und Stickstoffatmosphäre 0,86 g (0,02 Mol) einer 57prozentigen Dispersion von Natriumhydrid in Mineralöl, wobei eine starke Wasserstoffgasentwicklung stattfindet. Man lässt während 30 Minuten bei Raumtemperatur ausreagieren und versetzt dann mit 5,0 g (0,02 Mol) N-(Brom-acetyl)-glycyl-glycinamid. Das Reaktionsgemisch wird während 15 Stunden bei Raumtemperatur gerührt. Zur Aufarbeitung wird das Dimethylformamid im Hochvakuum eingedampft und der Rückstand in 250 ml heissem Ethanol digeriert. Man nutscht von den unlöslichen anorganischen Salzen ab und engt das Filtrat im Wasserstrahlvakuum bis zur beginnenden Kristallisation ein. Das ausgefallene, in Beispiel 1 beschriebene N-[(2-Oxo-1-pyrrolidinyl)-acetyl]-glycyl-glycinamid wird abgenutscht und mit wenig Ethanol nachgewaschen.

Beispiel 9: 2,0 g (0,01 Mol) N-[(2-Oxo-1-pyrrolidinyl)-acetyl]-glycin und 1,1 g (0,01 Mol) Glycinamid-hydrochlorid werden in 20 ml Dimethylformamid unter Rühren und Stickstoffatmosphäre nacheinander mit 1,1 g (0,011 Mol) Triethylamin und 3,26 g (0,0105 Mol) Triphenylphosphit versetzt. Hierauf wird das Reaktionsgemisch während 3 Stunden auf 85-90° erwärmt, wobei eine klare Lösung ensteht. Dann kühlt man auf 5° ab und versetzt mit 30 ml Diethylether. Der Niederschlag wird abgenutscht und zur Abtrennung von Triethylamin-hydrochlorid in 20 ml Ethanol aufgeschlämmt und wieder abgenutscht, wobei man das in Beispiel 1 beschriebene N-[(2-Oxo-1-pyrrolidinyl)-acetyl]-glycyl-glycinamid erhält.

Beispiel 10: 2,7 g (0,01 Mol) N-[(2-Oxo-1-pyrrolidinyl)-acetyl]-glycyl-glycin-methylester werden in 100 ml Methanol gelöst und unter Rühren wird Ammoniakgas eingeleitet. Man lässt insgesamt während 15 Stunden bei Raumtemperatur reagieren, nutscht dann das ausgefallene, in Beispiel 1 beschriebene N-[(2-Oxo-1-pyrrolidinyl)-acetyl]-glycyl-glycinamid ab und kristallisiert dieses aus Methanol um.

Beispiel 11: In ein Chromatographiegefäss mit 2,5 cm Durchmesser werden 50 ml feucht aufgeschlämmtes AMBERLITE® IRA-400 (starkbasisches Anionenaustauscherharz auf Polystyrolbasis) in Chloridform eingetragen und mit einem Gemisch von 20 ml Triethylamin, 20 ml Ethanol und 60 ml Wasser überschichtet. Das Gemisch wird tropfenweise durchgelassen und dann mit destilliertem Wasser solange gespült bis das Eluat neutral ist.

Das auf diese Weise aktivierte und gewaschene Amberlite® IRA-400 wird in einen Rundkolben transferiert und das Wasser abdekantiert.

Eine wässrige Lösung (100 ml) von 2,38 g (0,01 Mol) N-[(2-Oxo-1-pyrrolidinyl)-acetyl]-glycin-N-cyanomethyl-amid wird mit dem aktivierten Anionenaustauscher unter magnetischem Rühren 5 Std. lang unter Rückfluss gekocht. Bei Raumtemperatur wird der Ionenaustauscher auf einer Nutsche abgesaugt und das klare wässrige Filtrat bei 60°/14 Torr bis zur Trockene eingedampft. Das im Rückstand zurückbleibende, in Beispiel 1 beschriebene N-[(2-Oxo-1-pyrrolidinyl)-acetyl]-glycyl-glycinamid wird durch Umkristallisation aus Methanol gereinigt.

Das Ausgangsprodukt erhält man aus N-[(2-Oxo-1-pyrrolidinyl)-acetyl]-glycin und Aminoacetonitril.

Beispiel 12: In analoger Weise, wie in dieser Anmeldung beschrieben ist, erhält man N-[(2-Oxo-1-pyrrolidinyl)-acetyl]-(L)-alanyl-(L)-alaninamid, $R_f$ = 0,35 (Chloroform: Methanol = 4:1), N-[(2-Oxo-1-pyrrolidinyl)-acetyl]-(D,L)-phenylglycyl-(D,L)-phenylglycinamid, $R_f$ = 0,45 (Chloroform: Methanol = 4:1), (D,L)-N-[2-(2-Oxo-1-pyrrolidinyl)-2-benzyl-acetyl]-glycyl-glycinamid, $R_f$ = 0,40 (Chloroform: Methanol = 4:1),und (D,L)-N-[2-(2-Oxo-1-pyrrolidinyl)-2-phenyl-acetyl]-glycyl-glycinamid, $R_f$ = 0,41 (Chloroform: Methanol = 4:1).

Patentansprüche für die Vertragsstaaten BE, CH, DE, FR, GB, IT, LU, NL und SE

1. Lactampeptide der Formel I,

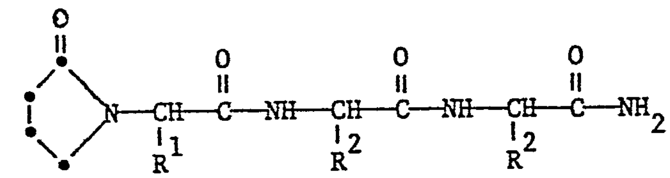

(I)

worin

$R^1$ Wasserstoff, $C_{1-4}$-Alkyl, Arylniederalkyl oder Aryl und

$R^2$ Wasserstoff, Niederalkyl oder Aryl bedeuten, wobei beide Reste $R^2$ die gleiche Bedeutung haben, und Salze von solchen Verbindungen mit mindestens einer salzbildenden Gruppe.

2. Verbindungen der Formel I nach Anspruch 1, worin $R^1$ Wasserstoff, $C_{1-4}$-Alkyl, durch einen unsubstituierten oder substituierten Phenyl- oder Naphthylrest substituiertes Niederalkyl oder einen unsubstituierten oder substituierten Phenylrest und $R^2$ Wasserstoff, Niederalkyl oder einen unsubstituierten oder substituierten Phenylrest bedeuten, und Salze von solchen Verbindungen mit mindestens einer salzbildenden Gruppe.

3. Verbindungen der Formel I nach Anspruch 1, worin $R^1$ Wasserstoff, unsubstituiertes oder durch Phenyl substituiertes $C_{1-4}$-Alkyl oder Phenyl und $R^2$ Wasserstoff, $C_{1-4}$-Alkyl, Phenyl oder 4-Hydroxy-phenyl bedeuten.

4. Verbindungen der Formel I nach Anspruch 1, worin $R^1$ und $R^2$ unabhängig voneinander Wasserstoff oder $C_{1-4}$-Alkyl bedeuten.

5. Verbindungen der Formel I nach Anspruch 1, worin $R^1$ Wasserstoff oder $C_{1-4}$-Alkyl und $R^2$ Wasserstoff, Methyl, Ethyl, 2-Propyl, 2-Butyl oder 2-Methyl-propyl bedeuten.

6. Verbindungen der Formel I nach einem der Ansprüche 1 bis 5, worin

$R^1$ Wasserstoff bedeutet und die an asymmetrisch substituierten C-$R^2$-Atomen die (L)-Kofiguration aufweisen.

7. Verbindungen der Formel I nach einem der Ansprüche 1 bis 5, die an asymmetrisch substituierten C-$R^2$-Atomen die (L)-Konfiguration aufweisen, sowie Salze von solchen Verbindungen mit mindestens einer salzbildenden Gruppe.

8. N-[(2-Oxo-1-pyrrolidinyl)-acetyl]-glycyl-glycinamid nach Anspruch 1.

9. Pharmazeutische Präparate, die eine Verbindung der Formel I nach einem der Ansprüche 1 bis 8 oder ein Salz einer solchen Verbindung mit mindestens einer salzbildenden Gruppe zusammen mit einem pharmazeutisch verwendbaren Trägermaterial enthalten.

10. Eine Verbindung gemäss einem der Ansprüche 1 bis 8 zur Verwendung in einem Verfahren zur therapeutischen Behandlung des menschlichen oder tierischen Körpers.

11. Verwendung einer Verbindung nach einem der Ansprüche 1 bis 8 zur Herstellung von pharmazeutischen Präparaten.

12. Verwendung einer Verbindung nach einem der Ansprüche 1 bis 8 zur Behandlung von zerebraler Leistungsinsuffizienz.

13. Verwendung einer Verbindung nach einem der Ansprüche 1 bis 8 zur Therapie von Tumorerkrankungen.

14. Verfahren zur Herstellung von Verbindungen der Formel I nach einem der Ansprüche 1 bis 8 und von Salzen von solchen Verbindungen, die mindestens eine salzbildende Gruppe enthalten, dadurch gekennzeichnet, dass man

a) eine Verbindung der Formel II,

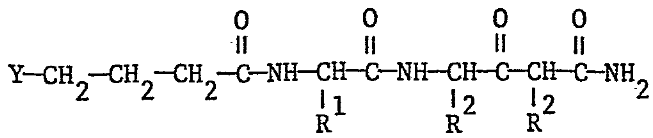

(II)

worin Y eine nucleophile Abgangsgruppe bedeutet und die übrigen Substituenten die obengenannten Bedeutungen haben, mit der Massgabe, dass in den Resten $R^1$ und $R^2$ gegebenenfalls vorhandene funktionelle Gruppen gegebenenfalls durch leicht abspaltbare Schutzgruppen geschützt sind, intramolekular unter Ausbildung des Pyrrolidonringes zyklisiert, oder

b) eine Verbindung der Formel III,

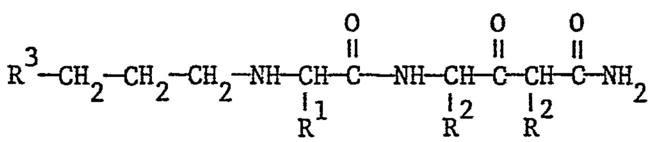

(III)

worin $R^3$ eine Carboxylgruppe oder ein aktiviertes Derivat derselben bedeutet und auch die an der Reaktion teilnehmende Aminogruppe in aktivierter Form vorliegen kann und die übrigen Substituenten die obengenannten Bedeutungen haben, mit der Massgabe, dass in den Resten $R^1$ und $R^2$ gegebenenfalls vorhandene funktionelle Gruppen gegebenenfalls durch leicht abspaltbare Schutzgruppen geschützt sind, intramolekular unter Ausbildung des Pyrrolidonringes zyklisiert oder

c) 2-Oxo-pyrrolidin der Formel IV

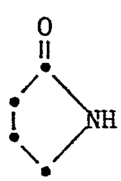

(IV)

oder eine reaktionsfähige Form dieser Verbindung mit einer Verbindung der Formel V,

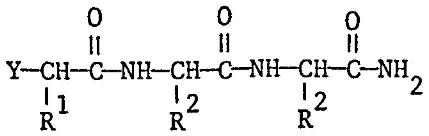 (V)

worin Y eine nucleophile Abgangsgruppe bedeutet und die übrigen Substituenten die obengenannten Bedeutungen haben, mit der Massgabe, dass in den Resten $R^1$ und $R^2$ vorhandene funktionelle Gruppen gegebenenfalls durch leicht abspaltbare Schutzgruppen geschützt sind, umsetzt, oder

d) eine Verbindung der Formel VI,

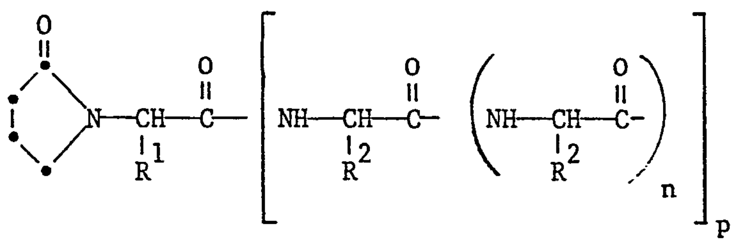 (VI)

worin n und p unabhängig voneinander die Zahl 0 oder 1 bedeuten, und die übrigen Substituenten die obengenannten Bedeutungen haben, mit der Massgabe, dass in den Resten $R^1$ und $R^2$ gegebenenfalls vorhandene funktionelle Gruppen gegebenenfalls durch leicht abspaltbare Schutzgruppen geschützt sind, oder ein reaktionsfähiges Carbonsäurederivat der Verbindung der Formel VI mit einer Verbindung der Formel VII,

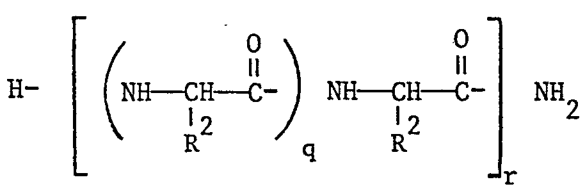 (VII)

worin q und r unabhängig voneinander für die Zahl 0 oder 1 stehen, mit der Massgabe, dass r und q jeweils für 1 stehen, wenn im Reaktionspartner der Formel VI p für 0 steht, oder dass r für 1 und q für 0 stehen, wenn p für 1 und n für 0 stehen, oder dass r für 0

steht, wenn p und n jeweils für 1 stehen, und worin die übrigen Substituenten die obengenannten Bedeutungen haben, mit der Massgabe, dass im Rest $R^2$ gegebenenfalls vorhandene funktionelle Gruppen gegebenenfalls durch leicht abspaltbare Schutzgruppen geschützt sind, oder ein Derivat der Verbindung der Formel VII, in dem eine der NH-Gruppen in reaktionsfähiger Form vorliegt, umsetzt, oder

e) die Cyanogruppe in einer Verbindung der Formel VIII ,

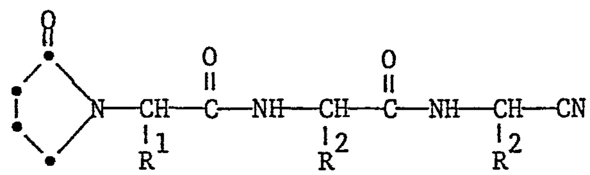

(VIII)

worin alle Substituenten die obengenannten Bedeutungen haben, in eine Amidgruppe überführt, oder

f) die Oximgruppe in einer Verbindung der Formel IX,

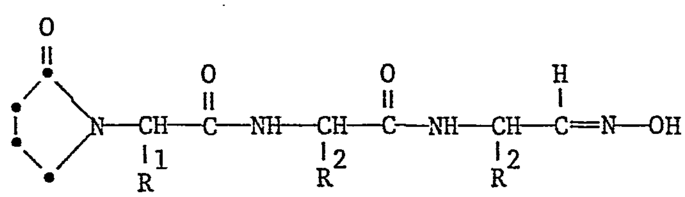

(IX)

worin die Substituenten die obengenannten Bedeutungen haben, durch eine Beckmann-Umlagerung in eine Amidgruppe überführt, oder

g) in einer Verbindung der Formel I, die mindestens eine freie Hydroxy- oder Carboxygruppe aufweist, freies Hydroxy verestert oder verethert oder freies Carboxy verestert und nach Ausführung der unter a) bis f) beschriebenen Verfahren gegebenenfalls vorhandene Schutzgruppen abspaltet und, wenn erwünscht, erhaltene Stereoisomerengemische auftrennt, und, wenn erwünscht, eine erhaltene Verbindung mit mindestens einer salzbildenden Gruppe in ein Salz oder ein erhaltenes Salz in die freie Verbindung überführt.

15. Die nach den Verfahren des Anspruchs 14 erhältlichen Verbindungen.

Patentansprüche für den Vertragsstaat AT

1. Verfahren zur Herstellung eines Lactampeptids der Formel I

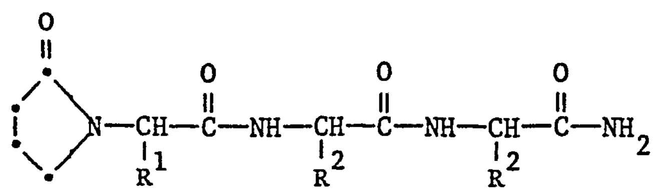 (I)

worin

$R^1$ Wasserstoff, $C_{1-4}$-Alkyl, Arylniederalkyl oder Aryl und

$R^2$ Wasserstoff, Niederalkyl oder Aryl bedeuten, wobei beide Reste $R^2$ die gleiche Bedeutung haben, oder eines Salzes einer solchen Verbindung mit mindestens einer salzbildenden Gruppe, dadurch gekennzeichnet, dass man

a) eine Verbindung der Formel II,

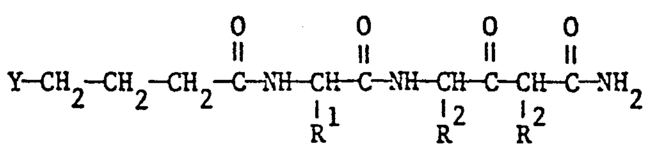 (II)

worin Y eine nucleophile Abgangsgruppe bedeutet und die übrigen Substituenten die obengenannten Bedeutungen haben, mit der Massgabe, dass in den Resten $R^1$ und $R^2$ gegebenenfalls vorhandene funktionelle Gruppen gegebenenfalls durch leicht abspaltbare Schutzgruppen geschützt sind, intramolekular unter Ausbildung des Pyrrolidonringes zyklisiert, oder

b) eine Verbindung der Formel III,

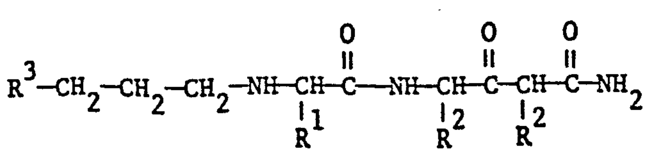 (III)

worin $R^3$ eine Carboxylgruppe oder ein aktiviertes Derivat derselben

bedeutet und auch die an der Reaktion teilnehmende Aminogruppe in aktivierter Form vorliegen kann und die übrigen Substituenten die obengenannten Bedeutungen haben, mit der Massgabe, dass in den Resten $R^1$ und $R^2$ gegebenenfalls vorhandene funktionelle Gruppen gegebenenfalls durch leicht abspaltbare Schutzgruppen geschützt sind, intramolekular unter Ausbildung des Pyrrolidonringes zyklisiert oder

c) 2-Oxo-pyrrolidin der Formel IV

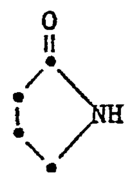

(IV)

oder eine reaktionsfähige Form dieser Verbindung mit einer Verbindung der Formel V,

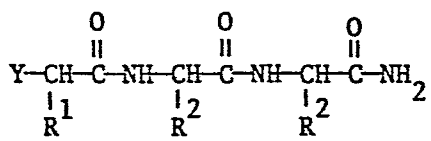

(V)

worin Y eine nucleophile Abgangsgruppe bedeutet und die übrigen Substituenten die obengenannten Bedeutungen haben, mit der Massgabe, dass in den Resten $R^1$ und $R^2$ vorhandene funktionelle Gruppen gegebenenfalls durch leicht abspaltbare Schutzgruppen geschützt sind, umsetzt, oder

d) eine Verbindung der Formel VI,

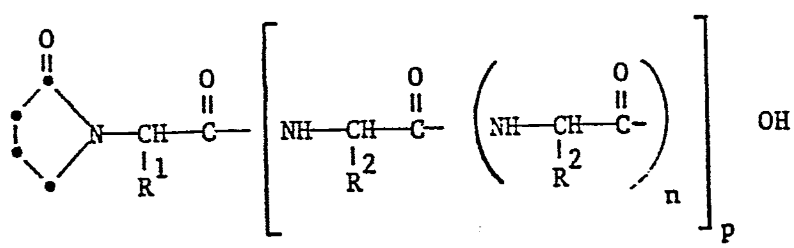

(VI)

worin n und p unabhängig voneinander die Zahl 0 oder 1 bedeuten, und die übrigen Substituenten die obengenannten Bedeutungen haben, mit

der Massgabe, dass in den Resten $R^1$ und $R^2$ gegebenenfalls vorhandene funktionelle Gruppen gegebenenfalls durch leicht abspaltbare Schutzgruppen geschützt sind, oder ein reaktionsfähiges Carbonsäurederivat der Verbindung der Formel VI mit einer Verbindung der Formel VII,

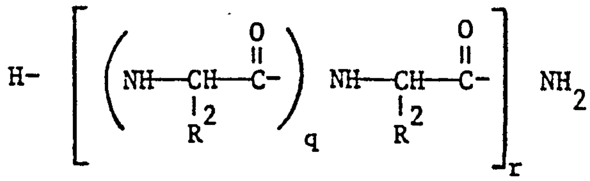

(VII)

worin q und r unabhängig voneinander für die Zahl 0 oder 1 stehen, mit der Massgabe, dass r und q jeweils für 1 stehen, wenn im Reaktionspartner der Formel VI p für 0 steht, oder dass r für 1 und q für 0 stehen, wenn p für 1 und n für 0 stehen, oder dass r für 0 steht, wenn p und n jeweils für 1 stehen, und worin die übrigen Substituenten die obengenannten Bedeutungen haben, mit der Massgabe, dass im Rest $R^2$ gegebenenfalls vorhandene funktionelle Gruppen gegebenenfalls durch leicht abspaltbare Schutzgruppen geschützt sind, oder ein Derivat der Verbindung der Formel VII, in dem eine der NH-Gruppen in reaktionsfähiger Form vorliegt, umsetzt, oder

e) die Cyanogruppe in einer Verbindung der Formel VIII ,

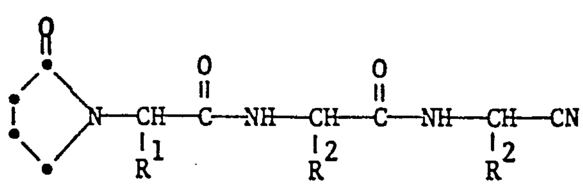

(VIII)

worin alle Substituenten die obengenannten Bedeutungen haben, in eine Amidgruppe überführt, oder

f) die Oximgruppe in einer Verbindung der Formel IX,

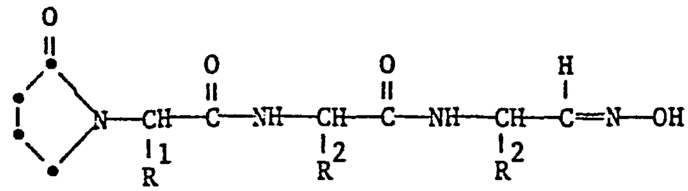

(IX)

worin die Substituenten die obengenannten Bedeutungen haben, durch eine Beckmann-Umlagerung in eine Amidgruppe überführt, oder

g) in einer Verbindung der Formel I, die mindestens eine freie Hydroxy- oder Carboxygruppe aufweist, freies Hydroxy verestert oder verethert oder freies Carboxy verestert und nach Ausführung der unter a) bis f) beschriebenen Verfahren gegebenenfalls vorhandene Schutzgruppen abspaltet und, wenn erwünscht, erhaltene Stereoisomerengemische auftrennt, und, wenn erwünscht, eine erhaltene Verbindung mit mindestens einer salzbildenden Gruppe in ein Salz oder ein erhaltenes Salz in die freie Verbindung überführt.

2. Verfahren nach Anspruch 1, dadurch gekennzeichnet, dass man die Ausgangsstoffe so wählt, dass man eine Verbindung der Formel I, worin $R^1$ Wasserstoff, $C_{1-4}$-Alkyl, durch einen unsubstituierten oder substituierten Phenyl- oder Naphthylrest substituiertes Niederalkyl oder einen unsubstituierten oder substituierten Phenylrest und $R^2$ Wasserstoff, Niederalkyl oder einen unsubstituierten oder substituierten Phenylrest bedeuten, oder ein Salz einer solchen Verbindung mit mindestens einer salzbildenden Gruppe erhält.

3. Verfahren nach Anspruch 1, dadurch gekennzeichnet, dass man die Ausgangsstoffe so wählt, dass man eine Verbindung der Formel I, worin $R^1$ Wasserstoff, unsubstituiertes oder durch Phenyl substituiertes $C_{1-4}$-Alkyl oder Phenyl und $R^2$ Wasserstoff, $C_{1-4}$-Alkyl, Phenyl oder 4-Hydroxy-phenyl bedeuten, erhält.

4. Verfahren nach Anspruch 1, dadurch gekennzeichnet, dass man die Ausgangsstoffe so wählt, dass man eine Verbindung der Formel I, worin $R^1$ und $R^2$ unabhängig voneinander Wasserstoff oder $C_{1-4}$-Alkyl bedeuten, erhält.

5. Verfahren nach Anspruch 1, dadurch gekennzeichnet, dass man die Ausgangsstoffe so wählt, dass man eine Verbindung der Formel I, worin $R^1$ Wasserstoff oder $C_{1-4}$-Alkyl und $R^2$ Wasserstoff, Methyl, Ethyl, 2-Propyl, 2-Butyl oder 2-Methyl-propyl bedeuten, erhält.

6. Verfahren nach einem der Ansprüche 1 bis 5, dadurch gekennzeichnet, dass man die Ausgangsstoffe so wählt, dass man eine Verbindung der Formel I, worin $R^1$ Wasserstoff bedeutet und die an asymmetrisch substituierten $\underline{C}$-$R^2$-Atomen die (L)-Konfiguration aufweist, erhält.

7. Verfahren nach einem der Ansprüche 1 bis 5, dadurch gekennzeichnet, dass man die Ausgangsstoffe so wählt, dass man eine Verbindung der Formel I, die an asymmetrisch substituierten $\underline{C}$-$R^2$-Atomen die (L)-Konfiguration aufweist, oder ein Salz einer solchen Verbindung mit mindestens einer salzbildenden Gruppe erhält.

8. Verfahren nach Anspruch 1, dadurch gekennzeichnet, dass man die Ausgangsstoffe so wählt, dass man N-[(2-Oxo-1-pyrrolidinyl)-acetyl]-glycyl-glycinamid erhält.